(19) Europäisches Patentamt
European Patent Office
Office européen des brevets

(11) Veröffentlichungsnummer: **0 174 653**
**A2**

(12) # EUROPÄISCHE PATENTANMELDUNG

(21) Anmeldenummer: **85111493.4**

(22) Anmeldetag: **11.09.85**

(51) Int. Cl.[4]: **C 07 D 211/90**
A 61 K 31/44

(30) Priorität: **14.09.84 CH 4388/84**

(43) Veröffentlichungstag der Anmeldung:
**19.03.86 Patentblatt 86/12**

(84) Benannte Vertragsstaaten:
**AT BE CH DE FR GB IT LI LU NL SE**

(71) Anmelder: **CIBA-GEIGY AG**
**Klybeckstrasse 141**
**CH-4002 Basel(CH)**

(72) Erfinder: **Zimmermann, Markus, Dr.**
**Steinbrecheweg 8**
**CH-4125 Riehen(CH)**

(74) Vertreter: **Zumstein, Fritz, Dr. et al,**
**Bräuhausstrasse 4**
**D-8000 München 2(DE)**

(54) **Phenylalkylverbindungen.**

(57) Verbindungen der Formel

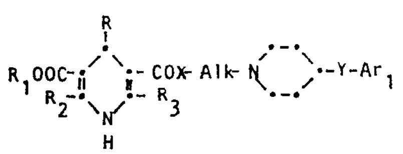

(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyan oder Amino steht, X Sauerstoff oder die Gruppe $-NH-$ darstellt, und Alk Niederalkylen bedeutet, das die Gruppe X vom Ringstickstoffatom durch mindestens zwei Kohlenstoffatome trennt, Y für Alkylen mit 1 bis 3 Kohlenstoffatomen steht und $Ar_1$ einen unsubstituierten oder substituierten Phenylrest darstellt sowie Salze davon, zeigen cardiovaskuläre, insbesondere coronardilatatorische und antihypertensive Eigenschaften.

EP 0 174 653 A2

0174653



CIBA-GEIGY AG
Basel (Schweiz)

4-15451/15072

Phenylalkylverbindungen

Die Erfindung betrifft neue Phenylalkylverbindungen und deren Salze, Verfahren zu ihrer Herstellung, diese enthaltende pharmazeutische Präparate sowie deren Verwendung.

Die erfindungsgemässen Verbindungen entsprechen der Formel

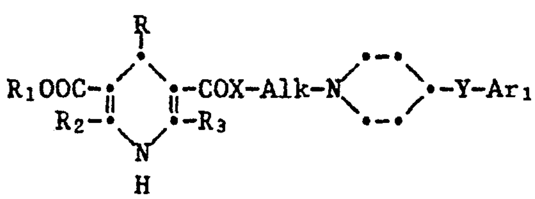

(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyan oder Amino steht, X Sauerstoff oder die Gruppe -NH- darstellt, und Alk Niederalkylen bedeutet, das die Gruppe X vom Ringstickstoffatom durch mindestens zwei Kohlenstoffatome trennt, Y für Alkylen mit 1 bis 3 Kohlenstoffatomen steht, und $Ar_1$ einen unsubstituierten oder substituierten Phenylrest darstellt, sowie Salze von Verbindungen der Formel I.

Ein carbocyclischer oder heterocyclischer Arylrest R ist in erster Linie ein entsprechender monocyclischer Rest, kann jedoch auch ein bi- oder polycyclischer, carbocyclischer oder heterocyclischer Rest mit aromatischen Eigenschaften sein.

Carbocyclische Reste R dieser Art sind insbesondere gegebenenfalls substituiertes Phenyl, ferner Naphthyl.

Heterocyclische Arylreste R sind vorzugsweise entsprechende monocyclische Reste, können jedoch auch entsprechende bi- oder polycyclische Reste sein, wobei letztere aus mehreren heterocyclischen Ringen, oder aus einem oder mehreren Ringen mit einem oder mehreren ankondensierten carbocyclischen Ringen, insbesondere einem oder mehreren ankondensierten Benzoringen bestehen können. Die üblicherweise vorliegenden heterocyclischen Reste R, die vorzugsweise aus fünf oder sechs Ringgliedern bestehen, können bis zu vier, gleiche oder verschiedene Hetero-, insbesondere Stickstoff-, Sauerstoff- und/oder Schwefelatome, vorzugsweise ein, zwei, drei oder vier Stickstoffatome, ein Sauerstoff- oder Schwefelatom, oder ein oder zwei Stickstoffatome zusammen mit einem Sauerstoff- oder Schwefelatom als Ringglieder enthalten. Sie sind mit dem Kohlenstoffatom der 4-Stellung des 1,4-Dihydropyridinringes üblicherweise über ein Ringkohlenstoffatom gebunden.

Monocyclische fünfgliedrige Heteroarylreste R sind z.B. entsprechende monoaza-, diaza-, triaza-, tetraza-, monooxa-, monothia-, oxaza-, oxadiaza-, thiaza- oder thiadiazacyclische Reste, wie Pyrryl-, Pyrazolyl-, Imidazolyl-, Triazolyl-, Tetrazolyl-, Furyl-, Thienyl-, Isoxazolyl-, Oxazolyl-, Oxadiazolyl-, Isothiazolyl-, Thiazolyl- oder Thiadiazolylreste, während monocyclische, sechsgliedrige Heteroarylreste R z.B. entsprechende monoaza-, diaza- oder triazacyclische Reste, wie Pyridyl-, Pyridazinyl-, Pyrimidinyl-, Pyrazinyl- oder Triazinylreste sind. Bicyclische Heteroarylreste sind insbesondere monocyclische Heteroarylreste mit ankondensiertem Benzoring; der Heteroring kann fünf- oder sechsgliedrig sein, wobei ein fünfgliedriger Heteroarylrest z.B. einen monoaza-, diaza-,

diaza-monooxa-, monooxa-, monothia-, oxaza- oder thiazacyclischen Rest, und ein sechsgliedriger Heteroarylrest z.B. einen monoaza-oder einen diazacyclischen Heteroarylrest darstellt. Solche bicyclischen Reste, die über ein Ringkohlenstoffatom des hetero- oder carbocyclischen Restes gebunden sein können, sind z.B. Indolyl-, Isoindolyl-, Benzimidazolyl-, Benzoxadiazolyl-, Benzofuranyl-, Benzofurazanyl-, Benzothienyl-, Benzthiazolyl-, Benzthiadiazolyl-, Chinolinyl- oder Isochinolinylreste, wobei stickstoffhaltige mono-oder bicyclische Heteroarylreste der genannten Art, insbesondere unsubstituierte oder substituierte Pyridylreste, auch als N-Oxide vorliegen können, z.B. Pyridyl-N-oxid.

Die carbocyclischen und heterocyclischen Arylreste R sowie der Phenylrest $Ar_1$ können unsubstituiert oder substituiert sein, wobei in erster Linie Ringkohlenstoffatome, aber auch Ringstickstoffatome substituiert, ein- bis dreifach vorhanden und gleich oder verschieden, vorzugsweise jedoch einfach vorhanden sein können. Substituenten von Ringkohlenstoffatomen sind u.a. gegebenenfalls substituierte Kohlenwasserstoffreste, wie entsprechende aliphatische, cycloaliphatische, aromatische oder araliphatische Kohlenwasserstoffreste, z.B. Niederalkyl, Niederalkenyl, Niederalkinyl, Niederalkylen, Cycloalkyl, Cycloalkylniederalkyl, Phenyl oder Phenylniederalkyl. Substituenten von solchen Kohlenwasserstoffresten, insbesondere von Niederalkyl, Phenyl oder Phenylniederalkyl, sind z.B. gegebenenfalls verätherte oder veresterte Hydroxygruppen, wie Hydroxy, gegebenenfalls, z.B. durch gegebenenfalls veräthertes oder verestertes Hydroxy substituiertes Niederalkoxy, z.B. Niederalkoxy, Niederalkoxyniederalkoxy, oder Halogenniederalkoxy, gegebenenfalls, z.B. durch gegebenenfalls veräthertes oder verestertes Hydroxy, substituiertes Niederalkenyloxy, z.B. Niederalkenyloxy oder Halogenniederalkenyloxy, Niederalkinyloxy, Niederalkylendioxy, Niederalkanoyloxy oder Halogen, und/oder gegebenenfalls funktionell abgewandeltes Carboxy, wie Carboxy, verestertes Carboxy, z.B. Niederalkoxycarbonyl, amidiertes Carboxy, wie Carbamoyl, N-Niederalkyl-carbamoyl oder N,N-Diniederalkyl-carbamoyl, oder Cyan. Cyclische Substituenten, insbesondere Phenyl, können zudem auch

Niederalkyl, das gegebenenfalls, z.B. wie angegeben, substituiert sein kann, als Substituenten enthalten. Weitere Substituenten von Arylresten R sowie $Ar_1$ sind z.B. gegebenenfalls verätherte oder veresterte Hydroxygruppen, wie Hydroxy, gegebenenfalls, z.B. durch gegebenenfalls veräthertes oder verestertes Hydroxy substituiertes Niederalkoxy, z.B. Niederalkoxy, Niederalkoxyniederalkoxy oder Halogenniederalkoxy, gegebenenfalls, z.B. durch gegebenenfalls veräthertes oder verestertes Hydroxy substituiertes Niederalkenyloxy, z.B. Niederalkenyloxy oder Halogenniederalkenyloxy, Niederalkinyloxy, Niederalkylendioxy, Niederalkanoyloxy oder Halogen, Nitro, gegebenenfalls substituiertes Amino, wie Amino, N-Niederalkylamino, N,N-Diniederalkylamino, N-Niederalkyl-N-phenylniederalkylamino, Niederalkylenamino, Oxaniederalkylenamino, Thianiederalkylenamino oder Azaniederalkylenamino, wobei der Aza-Stickstoff unsubstituiert oder, z.B. durch gegebenenfalls, z.B. wie oben beschrieben, substituiertes Niederalkyl, Phenyl oder Phenylniederalkyl substituiert sein kann, oder Acylamino, z.B. Niederalkanoylamino, Azido, Acyl, wie Niederalkanoyl oder gegebenenfalls funktionell abgewandeltes Carboxy, wie Carboxy, verestertes Carboxy, z.B. Niederalkoxycarbonyl, oder amidiertes Carboxy, wie Carbamoyl, N-Niederalkyl-carbamoyl oder N,N-Diniederalkyl-carbamoyl, ferner Cyan, gegebenenfalls funktionell abgewandeltes Sulfo, wie Sulfo, Aminosulfonyl, N-Niederalkylaminosulfonyl oder N,N-Diniederalkylaminosulfonyl und/oder veräthertes Mercapto, das gegebenenfalls oxidiert sein kann, wie Niederalkylthio, Niederalkylsulfinyl oder Niederalkylsulfonyl. Substituenten von Ringstickstoffatomen sind in erster Linie die obengenannten, gegebenenfalls substituierten Kohlenwasserstoffreste, wie Niederalkyl, ferner Hydroxy oder Oxido.

Heterocyclische Arylreste R können, z.B. je nach Art der Substitution, in verschiedenartigen tautomeren Formen vorliegen.

Der Alkylenrest Alk trennt die Gruppe X vom Ringstickstoffatom mindestens durch 2, vorzugsweise durch 2 bis 8 Kohlenstoffatome und ist z.B. Aethylen, 1,2- oder 1,3-Propylen, 1,4-Butylen, 1,5-Pentylen, oder 1,6-Hexylen, 1,7-Pentylen, oder 1,8-Octylen.

Der Alkylenrest Y mit 1 bis 3 Kohlenstoffatomen ist z.B. Methylen, Aethylen, 1,3-Propylen oder 1-Methyläthylen.

Die vor-, sowie nachstehend verwendeten Definitionen haben, sofern nicht besonders definiert, die folgenden Bedeutungen:

Der Ausdruck "nieder" bedeutet, dass entsprechend definierte Gruppen oder Verbindungen, sofern nicht anders definiert, bis und mit 7, vorzugsweise bis und mit 4, Kohlenstoffatome enthalten.

Substituierte Reste können einen oder mehrere, gleiche oder verschiedene Substituenten enthalten; diese können irgendeine geeignete Stellung einnehmen.

Naphthyl kann 1- oder 2-Naphthyl sein.

Pyrryl ist z.B. 2- oder 3-Pyrryl, Pyrazolyl z.B. 3- oder 4-Pyrazolyl, Imidazolyl z.B. 2- oder 4-Imidazolyl, Triazolyl z.B. 1,3,5-1H-Triazol-2-yl oder 1,3,4-Triazol-2-yl, und Tetrazolyl z.B. 1,2,3,4-1H-Tetrazol-5-yl, während Furyl 2- oder 3-Furyl und Thienyl 2- oder 3-Thienyl bedeuten. Isoxazolyl ist z.B. 3-Isoxazolyl, Oxazolyl z.B. 2- oder 4- Oxazolyl, Oxadiazolyl z.B. 1,3,4-Oxadiazol-2-yl, Isothiazolyl z.B. 3-Isothiazolyl, Thiazolyl 2- oder 4-Thiazolyl, und Thiadiazolyl z.B. 1,3,4-Thiadiazolyl-2-yl.

Pyridyl ist 2-, 3- oder 4-Pyridyl, Pyridazinyl z.B. 3-Pyridazinyl, Pyrimidinyl 2-, 4- oder 5-Pyrimidinyl, Pyrazinyl, 2-Pyrazinyl und Triazinyl z.B. 1,3,5-Triazin-2-yl.

Indolyl ist z.B. 2-, 3- oder 5-Indolyl, Isoindolyl z.B. 1-Isoindolyl, Benzimidazolyl z.B. 2- oder 5-Benzimidazolyl, Benzofuranyl z.B. 2- oder 3-Benzofuranyl, Benzofurazanyl (auch 2,1,3-Benzoxadiazolyl) z.B. 4-Benzofurazanyl, Benzothienyl z.B. 3-Benzothienyl, Benzthiazolyl z.B. 2-Benzthiazolyl, 2,1,3-Benzthiadiazolyl z.B. 2,1,3-Benzthiadiazol-4-yl, Chinolinyl z.B. 2- oder 4-Chinolinyl, und Isochinolinyl z.B. 1-Isochinolinyl.

Niederalkyl ist z.B. Methyl, Aethyl, n-Propyl, Isopropyl, n-Butyl, Isobutyl oder tert.-Butyl, ferner n-Pentyl, n-Hexyl oder n-Heptyl, während Niederalkenyl z.B. Allyl oder Methallyl, und Niederalkinyl z.B. Propargyl bedeutet.

Cycloalkyl weist vorzugsweise 5 bis 7 Ringkohlenstoffatome auf und ist z.B. Cyclopentyl oder Cyclohexyl, während Cycloalkylniederalkyl z.B. Cyclopropylmethyl, Cyclopentylmethyl oder Cyclohexylmethyl sein kann.

Phenylniederalkyl ist z.B. Benzyl oder 1- oder 2-Phenyläthyl.

Niederalkoxy steht in erster Linie für Methoxy, Aethoxy, n-Propyloxy, Isopropyloxy, n-Butyloxy, Isobutyloxy oder tert.-Butyloxy.

In einem Niederalkoxyniederalkoxyrest ist die terminale Niederalkoxygruppe vorzugsweise durch mehr als ein Kohlenstoffatom vom Verknüpfungssauerstoffatom getrennt; solche Reste sind z.B. 2-Methoxyäthoxy oder 2-Aethoxy-äthoxy.

In einem Halogen-niederalkoxyrest können ein oder mehrere Halogenatome, die vorzugsweise eine Atomnummer bis 35 aufweisen und besonders für Fluor und/oder Chlor stehen, vorhanden sein; solche Reste sind z.B. Difluormethoxy oder 1,1,2-Trifluor-2-chlor-äthoxy.

Niederalkenyloxy ist z.B. Allyloxy oder Methallyloxy, und Halogenniederalkenyloxy, das ein oder mehrere Halogenatome enthalten kann, wobei letztere vorzugsweise eine Atomnummer bis 35 aufweisen und besonders für Fluor und/oder Chlor stehen, ist z.B. 1,2-Dichlor-vinyloxy.

Niederalkinyloxy ist z.B. Propargyloxy, während Niederalkylendioxy z.B. Methylendioxy oder Aethylendioxy bedeutet.

Niederalkylen ist z.B. 1,2-Aethylen oder 1,3-Propylen, während Niederalkylendioxy z.B. Methylendioxy oder 1,2-Aethylendioxy ist.

Niederalkanoyloxy ist z.B. Acetyloxy, Propionyloxy oder Pivaloyloxy.

Halogen hat vorzugsweise eine Atomnummer bis und mit 35 und ist insbesondere Fluor oder Chlor, ferner Brom, kann jedoch auch Jod sein.

Halogensubstituiertes Niederalkyl ist z.B. Trifluormethyl, 1,1,2-Trifluor-2-chlor-äthyl oder Chlormethyl.

Niederalkoxycarbonyl ist z.B. Methoxycarbonyl, Aethoxycarbonyl, n-Propyloxycarbonyl, Isopropyloxycarbonyl, n-Butyloxycarbonyl, Isobutyloxycarbonyl oder tert.-Butyloxycarbonyl.

N-Niederalkyl-carbamoyl ist z.B. N-Methyl-carbamoyl oder N-Aethylcarbamoyl, während N,N-Diniederalkyl-carbamoyl z.B. N,N-Dimethyl-carbamoyl oder N,N-Diäthyl-carbamoyl ist.

N-Niederalkylamino ist z.B. N-Methylamino, N-Aethylamino, N-n-Propylamino oder N-Isopropylamino.

N,N-Diniederalkylamino ist z.B. N,N-Dimethylamino, N-Aethyl-N-methylamino oder N,N-Diäthylamino, während N-Niederalkyl-N-phenylniederalkylamino z.B. N-Benzyl-N-methylamino oder N-methyl-N-(2-phenyläthyl)-amino darstellt.

Niederalkylenamino enthält vorzugsweise 4 bis 6 Ringkohlenstoffatome und ist z.B. Pyrrolidino oder Piperidino, während Oxaniederalkylenamino z.B. 4-Morpholino, Thianiederalkylenamino z.B. 4-Thiomorpholino, und gegebenenfalls azasubstituiertes Azaniederalkylenamino z.B. Piperazino, 4-Methylpiperazino, 4-Phenyl-piperazino, 4-Benzylpiperazino oder 4-(2-Phenyläthyl)-piperazino darstellen kann.

Niederalkanoylamino ist z.B. Acetylamino oder Propionylamino.

Niederalkanoyl ist z.B. Formyl, Acetyl, Propionyl oder Pivaloyl.

Niederalkylthio ist z.B. Methylthio, Aethylthio, n-Propylthio oder Isopropylthio, während Niederalkylsulfinyl z.B. Methylsulfinyl, und Niederalkylsulfonyl z.B. Methylsulfonyl oder Aethylsulfonyl bedeuten.

N-Niederalkylaminosulfonyl ist z.B. N-Methylaminosulfonyl, während N,N-Diniederalkylaminosulfonyl z.B. N,N-Dimethylaminosulfonyl ist.

In einem substituierten Niederalkoxycarbonyl ist der Substituent üblicherweise durch mindestens 2, vorzugsweise durch 2 oder 3 Kohlenstoffatome vom Sauerstoff getrennt. Solche Reste sind z.B. Hydroxyniederalkoxycarbonyl, wie 2-Hydroxyäthoxycarbonyl oder 2,3-Dihydroxypropyloxycarbonyl, Niederalkoxy-niederalkoxycarbonyl, z.B. 2-Methoxyäthoxycarbonyl, Diniederalkylamio-niederalkoxycarbonyl, z.B. 2-Dimethylaminoäthoxycarbonyl, 2-Diäthylaminoäthoxycarbonyl oder 3-Dimethylaminopropyloxycarbonyl, Niederalkylenaminoniederalkoxycarbonyl, z.B. 2-Pyrrolidinoäthoxycarbonyl oder 2-Piperidinoäthoxycarbonyl, Morpholino-niederalkoxycarbonyl, z.B. 2-(4-Morpholino)-äthoxycarbonyl, oder (4-Niederalkyl-piperazino)-n-niederalkoxycarbonyl, z.B. 2-(4-Methylpiperazino)-äthoxycarbonyl.

Phenylniederalkoxycarbonyl ist z.B. Benzyloxycarbonyl oder 2-Phenyläthoxy-carbonyl.

N,N-Niederalkylen-carbamoyl ist z.B. Pyrrolidinocarbonyl oder Piperidinocarbonyl, wobei entsprechende Reste, in welchen der Niederalkylenteil durch Sauerstoff, Schwefel oder unsubstituierten

oder substituierten Stickstoff unterbrochen ist, z.B. 4-Morpholinocarbonyl, 4-Thiomorpholinocarbonyl, 1-Piperazinocarbonyl oder 4-Methyl-1-piperazinocarbonyl bedeutet.

Verbindungen der Formel I können in Form von Salzen, insbesondere von Säureadditionssalzen, in erster Linie entsprechenden pharmazeutisch verwendbaren, nicht-toxischen Säureadditionssalzen, vorliegen. Solche Salze sind z.B. diejenigen mit Halogenwasserstoffsäuren, z.B. Chlorwasserstoffsäure oder Bromwasserstoffsäure, ferner Salpetersäure, Schwefelsäure oder Phosphorsäure, oder organischen Säuren, wie Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren, oder organischen Sulfonsäuren, wie gegebenenfalls Hydroxy enthaltende Niederalkansulfonsäuren, z.B. Methansulfonsäure, Aethansulfonsäure, 2-Hydroxyäthansulfonsäure oder Aethan-1,2-disulfonsäure, oder Arylsulfonsäure, z.B. Benzolsulfonsäure, 4-Methyl-benzolsulfonsäure oder Naphthalin-2-sulfonsäure, oder mit anderen sauren organischen Substanzen, wie Ascorbinsäure.

Die Verbindungen der Formel I und deren Salze besitzen wertvolle pharmakologische Eigenschaften, vor allem im cardiovasculären Bereich. Sie wirken als Calcium-Antagonisten und besitzen α-Rezeptoren blockierende Eigenschaften, wie sich z.B. in in-vitro-Versuchen am isoliert perfundierten Mesenterialbett der Ratte [Mc Gregor D.D.: J.Physiol. 177, 21-30, (1965)] als Hemmung der Kalium- und Noradrenalin- induzierten Vasokonstriktion, beispielsweise unter Verwendung von 2,6-Dimethyl-4-(3-nitrophenyl)-1,4-dihydropyridin-3,5-dicarbonsäure-methylester-2-(4-benzyl-piperidino)-äthylester (Verbindung 1) oder von 2,6-Dimethyl-4-(3-nitrophenyl)-1,4-dihydropyridin-3,5-dicarbonsäure-methylester-2-[4-(2-phenäthyl)-piperidino]-äthylester (Verbindung 2) anhand der in der nachfolgenden Tabelle angegebenen Werte zeigen lässt, wobei zu

Vergleichszwecken der aus der Europa-Patentanmeldung 138.505 bekannte 2,6-Dimethyl-4-(3-nitrophenyl)-1,4-dihydropyridin-3,5-dicarbonsäure-methylester-2-(4-diphenylmethyl-piperidino)-äthylester (Verbindung 3) berücksichtigt ist.
Ferner lässt sich die Bindung der neuen Verbindungen oder deren Salze an Dihydropyridin-Rezeptoren mittels Verdrängung der Bindung von $^3H$-Nitrendipin an Membranen von Meerschweinchenherzen in in-vitro-Versuchen [Erne P. et al,: Biochem., Biophys. Res. Comm., 118, 842-847 (1984)] z.B. unter Verwendung von beispielsweise der oben definierten Verbindungen 1 und 2 nachweisen, wobei zu Vergleichszwecken die vorhin definierte bekannte Verbindung 3 berücksichtigt ist.

Tabelle

| | Verbindung 1 $IC_{50}$ nMol/Liter* | Verbindung 2 $IC_{50}$ nMol/Liter | Verbindung 3 $IC_{50}$ nMol/Liter |
|---|---|---|---|
| Hemmung der Kalium-induzierten vasokonstriktion | 6 | 9 | 6 |
| Hemmung der Noradrenalin-induzierten Vasokonstriktion | 0,7 | 1,7 | 12 |
| Verdrängung der Bindung von $^3H$-Nitrendipin | 2 | 0,4 | 5 |

*nMol/Liter = nano-Mol/Liter

Wie die angegebenen Werte erkennen lassen, ist bei den Verbindungen 1 und 2 hinsichtlich der Hemmung der Noradrenalin-induzierten Vasokonstriktion als auch der Verdrängung der Bindung von $^3H$-Nitrendipin eine deutlich ausgeprägte Ueberlegenheit gegenüber der bekannten Verbindung 3 erkennbar.

Die Verbindungen der Formel I und Salze von solchen Verbindungen weisen somit Eigenschaften von Calcium-Antagonisten ("Calcium-Entry-Blocker") und von Noradrenalin-Antagonisten (α-Rezeptoren-Blocker) auf und können daher z.B. als Coronardilatatoren und Antihypertensiva zur Behandlung von cardiovasculären Krankheitszuständen, wie Angina pectoris und ihre Folgen, Gefässspasmen, zentrale und periphere Durchblutungsstörungen, hohem Blutdruck, Arrhythmien und Herzinsuffizienz, ferner zur Hemmung der Plättchenaggregation Verwendung finden. Die neuen Verbindungen sind aber auch wertvolle Zwischenprodukte zur Herstellung anderer, insbesondere pharmazeutisch wirksamer Verbindungen.

Die Erfindung betrifft insbesondere neue Verbindungen der Formel I, in welchen R für einen mono- oder bicyclischen, carbocyclischen Arylrest oder für einen fünf- oder sechsgliedrigen, ein bis und mit vier Ringstickstoffatome, ein Ringsauerstoff- oder Ringschwefelatom, oder ein oder zwei Ringstickstoffatome zusammen mit einem Ringsauerstoff- oder einem Ringschwefelatom als Ringglieder enthaltenden, monocyclischen Heteroarylrest steht, der über ein Ringkohlenstoffatom mit dem Kohlenstoffatom der 4-Stellung des 1,4-Dihydropyridinrings verbunden ist, und der gegebenenfalls einen ankondensierten Benzoring enthält, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzoxadiazolyl, Benzofuranyl, Benzofurazanyl, Benzothienyl, Benzthiazolyl, 2,1,3-Benzthiadiazolyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl, wobei Niederalkyl, Phenyl oder Phenylniederalkyl gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, und cyclische Reste auch

Niederalkyl, das seinerseits wie angegeben substituiert sein kann, als Substituenten enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N-Diniederalkylamino, N-Niederalkyl-N-phenylniederalkyl-amino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan enthalten kann, oder durch Hydroxy oder Oxido substituiert sein können, $R_1$ Niederalkyl ist, einer der Reste $R_2$ und $R_3$ für Niederalkyl und der andere für Niederalkyl, Cyan oder Amino steht, X Sauerstoff oder die Gruppe -NH- bedeutet, und Alk Niederalkylen darstellt, das die Gruppe X vom Ringstickstoffatom durch 2 bis 8 Kohlenstoffatome trennt, Y für Alkylen mit 1 bis 3 Kohlenstoffatomen steht, und $Ar_1$ Phenyl bedeutet, welches gegebenenfalls durch Niederalkyl, Niederalkenyl, Niederalkinyl, Niederalkylen, Cycloalkyl, Phenyl und/oder Phenylniederalkyl substituiert ist, wobei Niederalkyl, Phenyl oder Phenylniederalkyl als Substituenten Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, und cyclische Reste auch Niederalkyl, das seinerseits wie angegeben substituiert sein kann, enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N-Diniederalkylamino, N-Niederalkyl-N-phenylniederalkyl-amino, Niederalkylenamino, Oxoniederalkylenmino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkylcarbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan als Substituenten enthalten kann, oder durch Hydroxy oder Oxido substituiert sein können, sowie Salze, insbesondere pharmazeutisch verwendbare, nichttoxische Säureadditionssalze solcher Verbindungen.

Die Erfindung betrifft in erster Linie neue Verbindungen der Formel I, worin R für Phenyl oder Naphthyl stehen und $Ar_1$ für Phenyl steht, wobei solche Reste R und $Ar_1$ jeweils gegebenenfalls durch

Niederalkyl, Phenyl und/oder Phenylniederalkyl, wobei solche Reste ihrerseits Hydroxy, Niederalkoxy, Halogenniederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogenniederalkoxy, Niederalkenyloxy, Halogenniederalkenyloxy, Niederalkylendioxy, Halogen, Nitro, Amino, N-Niederalkylamino, N,N-Diniederalkylamino, Niederalkanoylamino, Carboxy, Niederalkoxycarbonyl, Carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl substituiert sind, R zudem für über ein Ringkohlenstoffatom gebundenes Pyrryl, Furyl, Thienyl, Pyridyl, 1-Oxido-pyridyl, Imidazolyl, Benzofurazanyl oder Benzoxadiazolyl stehen kann, wobei solche Reste gegebenenfalls, wie für einen Phenyl- oder Naphthylrest R bzw. einen Phenylrest $Ar_1$ angegeben, substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten können, $R_1$ Niederalkyl ist, $R_2$ und $R_3$ jeweils Niederalkyl bedeuten, oder eine der Gruppen $R_2$ und $R_3$ Niederalkyl ist und die andere Niederalkyl oder Cyan darstellt, X vorzugsweise Sauerstoff oder die Gruppe -NH- bedeutet, und Alk für die Gruppe $-(CH_2)_n-$steht, worin n Werte von 2 bis 6 darstellt, und Y für Alkylen mit 1 bis 3 Kohlenstoffatomen steht, sowie Salze, insbesondere pharmazeutisch verwendbare, nichttoxische Säureadditionssalze solcher Verbindungen.

Die Erfindung betrifft ganz besonders neue Verbindungen der Formel I, worin R und $Ar_1$ jeweils für Phenyl steht, das gegebenenfalls durch Niederalkyl, z.B. Methyl, Hydroxy, Niederalkoxy, z.B. Methoxy oder Aethoxy, Halogenniederalkoxy, z.B. Difluormethoxy oder 2-Chlor-1,1,2-trifluoräthoxy, Halogenniederalkenyloxy, z.B. 1,2-Dichlor-vinyloxy, Niederalkylendioxy, z.B. Methylendioxy, Halogen, z.B. Fluor, Chlor oder Brom, Trifluormethyl, Nitro, Niederalkanoylamino, z.B. Acetylamino, gegebenenfalls durch gegebenenfalls verestertes oder veräthertes Hydroxy, wie Halogen, z.B. Fluor oder Chlor, oder Niederalkoxy,

z.B. Methoxy, substituiertes Phenyl oder Phenylniederalkyl, und/oder Cyan substituiert ist, wobei ein Phenylrest R bzw. $Ar_1$ einen oder mehrere, gleiche oder verschiedene Substituenten aufweisen kann, R zudem Pyridyl, z.B. 2-, 3- oder 4-Pyridyl, Furyl, z.B. 2-Furyl, 1-Oxido-pyridyl, z.B. 1-Oxido-3-pyridyl, Thienyl, z.B. 2-Thienyl, Benzofurazanyl, z.B. 4-Benzofurazanyl bedeuten kann, wobei solche Reste R durch Niederalkyl, z.B. Methyl, oder Halogen, z.B. Fluor oder Chlor, substituiert sein können, $R_1$, $R_2$ und $R_3$ jeweils Niederalkyl bedeuten, X insbesondere für Sauerstoff, ferner für die Gruppe -NH- steht, Alk die Gruppe $-(CH_2)_n-$ darstellt, worin n Werte von 2 bis 4 bedeutet, und Y für Alkylen mit 1 bis 3 Kohlenstoffatomen steht sowie Salze, insbesondere pharmazeutisch verwendbare nicht-toxische Säureadditonssalze solcher Verbindungen.

Die Erfindung betrifft insbesondere neue Verbindungen der Formel I, worin R für unsubstituiertes oder vorzugsweise durch Niederalkyl, z.B. Methyl, Niederalkoxy, z.B. Methoxy, Halogenniederalkoxy oder Halogenniederalkenyloxy, worin Halogen eine Atomnummer bis und mit 35 hat und insbesondere Fluor oder Chlor ist, z.B. Difluormethoxy, Halogen mit einer Atomnummer bis und mit 35, Trifluormethyl, Nitro und/oder Cyan mono- oder di-substituiertes Phenyl steht, wobei Substituenten die 2- und/oder 3-Stellung einnehmen, $R_1$, $R_2$ und $R_3$ jeweils Niederalkyl bedeuten, X insbesondere für Sauerstoff, ferner für die Gruppe -NH- steht, Alk die Gruppe $-(CH_2)_n-$ darstellt, worin n Werte von 2 bis 4 bedeutet, Y für Alkylen mit 1 bis 3, insbesondere 1 bis 2 Kohlenstoffatome steht, und $Ar_1$ gegebenenfalls durch Halogen mit einer Atomnummer bis und mit 35 substituiertes Phenyl bedeutet, sowie Salze, insbesondere pharmazeutisch verwendbare, nicht-toxische Säureadditionssalze solcher Verbindungen.

Die Erfindung betrifft insbesondere neue Verbindungen der Formel I, worin R für durch Halogen mit einer Atomnummer bis und mit 35, z.B. Fluor, Chlor oder Brom, mono- oder disubstituiertes oder durch Trifluormethyl, Nitro oder Cyan monosubstituiertes Phenyl steht, wobei Substituenten, die 2- und/oder 3-Stellung einnehmen, $R_1$ Niederalkyl, insbesondere Methyl oder Aethyl, und $R_2$ und $R_3$ jeweils

Niederalkyl, in erster Linie Methyl, bedeuten, X für Sauerstoff steht, Alk die Gruppe $-(CH_2)_n-$ darstellt, worin n Werte von 2 bis 3 bedeutet, Y für 1,3-Propylen, insbesondere Methylen oder 1,2-Aethylen steht, und $Ar_1$ unsubstituiertes oder durch Halogen mit einer Atomnummer bis und mit 35, z.B. Fluor, substituiertes Phenyl ist, sowie Salze, insbesondere pharmazeutisch verwendbare, nicht-toxische Säureadditionssalze solcher Verbindungen.

Die Erfindung betrifft insbesondere neue Verbindungen der Formel I, worin R für durch Halogen mit einer Atomnummer bis und mit 35, z.B. Fluor, Chlor oder Brom, mono- oder disubstituiertes, oder vorzugsweise durch Nitro, ferner durch Cyan monosubstituiertes Phenyl steht, wobei Substituenten die 2- und/oder 3-Stellung einnehmen, $R_1$ Niederalkyl, insbesondere Methyl oder Aethyl, und $R_2$ und $R_3$ jeweils Niederalkyl, in erster Linie Methyl, bedeuten, X für Sauerstoff steht, Alk die Gruppe $-(CH_2)_n-$ darstellt, worin n die Werte von 2 bis 3 bedeutet, Y für Methylen oder 1,2-Aethylen steht und $Ar_1$ unsubstituiertes oder durch Halogen mit einer Atomnummer bis und mit 35, besonders durch Fluor, in erster Linie in 4-Stellung substituiertes Phenyl ist, sowie Salze, insbesondere pharmazeutisch verwendbare, nicht-toxische Säureadditionssalze solcher Verbindungen.

Die Erfindung betrifft insbesondere die in den Beispielen beschriebenen spezifischen Verbindungen.

Die Verbindungen der Formel I und Salze von solchen Verbindungen mit salzbildenden Eigenschaften können in an sich bekannter Weise hergestellt werden, indem man z.B.

a) eine Verbindung der Formel

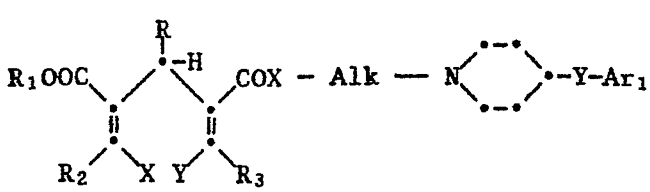

(II),

worin einer der Reste X und Y für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

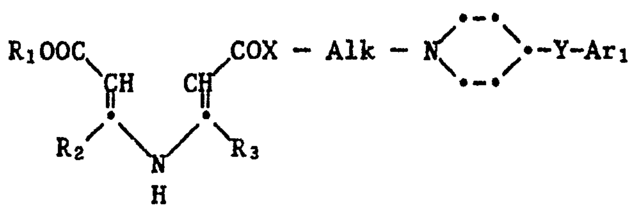

(IV)

oder einem Tautomeren davon oder einem entsprechenden Tautomerengemisch umsetzt, oder

c) in einer Verbindung der Formel

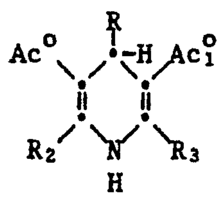

(V),

in welcher einer der Reste $Ac^o$ und $Ac_1^o$ eine in die Gruppe $-COOR_1$ oder in den Rest der Formel

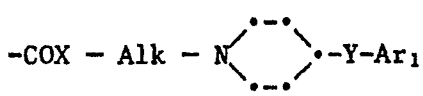

(Va)

überführbare Gruppe bedeutet, und der andere die Gruppe $-COOR_1$ oder die Gruppe der Formel Va bedeutet, den Rest $Ac^o$ bzw. $Ac_1^o$ in die Gruppe $-COOR_1$ bzw. in einen Rest der Formel Va überführt, oder

d) eine Verbindung der Formel

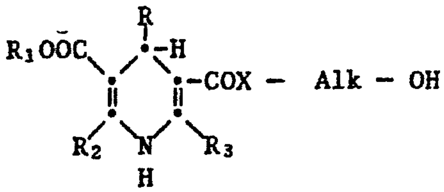

(VI)

oder einen reaktionsfähigen Ester davon, mit einer Verbindung der Formel

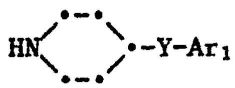

(VIa)

umsetzt, oder

e) eine Verbindung der Formel

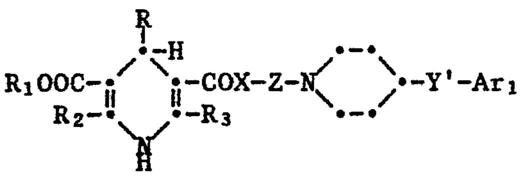

(VII)

worin Z die Bedeutung von Alk hat oder eine mittels Reduktion in die Gruppe Alk überführbare Gruppe darstellt, Y' die Bedeutung von Y hat oder eine mittels Reduktion in die Gruppe Y überführbare Gruppe darstellt, wobei mindestens eine der Gruppen Z und Y' von der Gruppe Alk oder Y verschieden ist, die Gruppe Z und/oder die Gruppe Y' zur Gruppe Alk und/oder zur Gruppe Y reduziert, wobei die Ausgangsstoffe der Formel II bis VII sofern sie reaktionsfähige Derivate bilden können oder salzbildende Eigenschaften aufweisen, auch in Form von reaktionsfähigen Derivaten, oder in Form ihrer Salze verwendet werden können, und die Gruppen R, $R_1$, $R_2$, $R_3$, X, Y, $Ar_1$ und Alk die unter der Formel I angegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder,

wenn erwünscht, eine erhaltene freie Verbindung der Formel I in ein Salz umwandelt, und/oder, wenn erwünscht, ein erhaltenes Isomerengemisch in die individuellen Isomere auftrennt.

Ueblicherweise werden die in der Verfahrensvariante a) verwendeten Ausgangsstoffe der Formel II in situ gebildet, und der verfahrensgemässe Ringschluss kann unter den Reaktionsbedingungen für die Herstellung des Ausgangsmaterials stattfinden. So kann man die Ausgangsstoffe der Formel II und unter den Reaktionsbedingungen üblicherweise auch die entsprechenden Endprodukte der Formel I erhalten, indem man aa) eine Verbindung der Formel III, oder ein reaktionsfähiges funktionelles Derivat davon, mit einer Verbindung der Formel

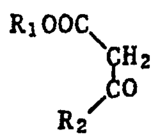

(VIII)

und einer Verbindung der Formel

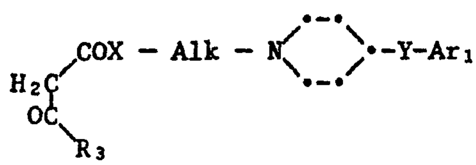

(IX),

und Ammoniak umsetzt, oder ab) eine Verbindung der Formel III oder ein reaktionsfähiges funktionelles Derivat davon mit einer Verbindung der Formel

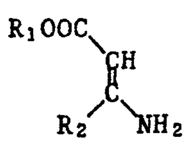

(X),

und einer Verbindung der Formel IX umsetzt, oder ac) eine Verbindung der Formel III oder ein reaktionsfähiges funktionelles Derivat davon mit einer Verbindung der Formel

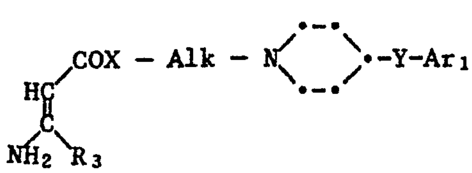

(XI),

und einer Verbindung der Formel VIII oder X umsetzt, oder ad) Ammoniak mit einer Verbindung der Formel

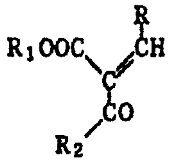

(XII)

und einer Verbindung der Formel IX umsetzt, oder ae) Ammoniak mit einer Verbindung der Formel

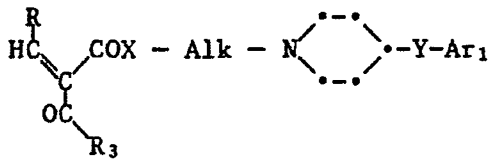

(XIII)

und einer Verbindung der Formel VIII oder X umsetzt, oder af) Ammoniak mit einer Verbindung der Formel

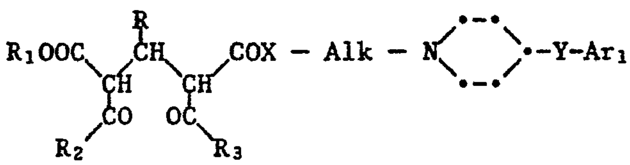

(XIV),

umsetzt, oder ag) eine Verbindung der Formel X mit einer Verbindung der Formel XIII oder der Formel

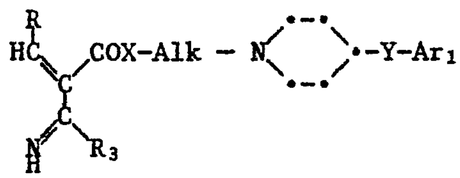

(XV),

umsetzt, oder ah) eine Verbindung der Formel XI mit einer Verbindung der Formel XII oder mit einer Verbindung der Formel

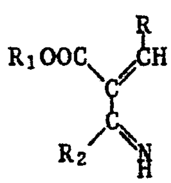

(XVI),

umsetzt. Dabei können mit Ausnahme der Verbindung der Formel III die Verbindungen der Formeln VIII bis XVI in Form von Tautomeren davon oder in Form von Tautomerengemischen verwendet werden; Ausgangsstoffe der obigen Formeln mit salzbildenden Eigenschaften können auch in Form von Salzen verwendet werden. Ferner haben in den obgenannten Verbindungen die Gruppen R, $R_1$, $R_2$, $R_3$, X, Y, $Ar_1$ und Alk die im Zusammenhang mit der Formel I gegebenen Bedeutungen.

Reaktionsfähige funktionelle Derivate des Aldehyds der Formel III sind u.a. die entsprechenden Acetale, d.h. die der Gruppe R entsprechenden Di-(veräthertes Hydroxy)-methyl-Verbindungen, wie Diniederalkyl-, z.B. Dimethyl- oder Diäthylacetale, Acylale, z.B. die entsprechenden Di-Acyloxymethyl- oder Di-Halogen-methyl-Verbindungen, wie Diniederalkanoylacylale, z.B. Diacetylacylale, oder die entsprechenden Dihalogen-, z.B. Dichlor- oder Dibrom-Verbindungen, ferner Additionsverbindungen, wie solche mit einem Alkalimetall-, z.B. Kaliumhydrogensulfit.

Das für die vorstehend beschriebenen Ringschlussreaktionen verwendete Ammoniak kann auch in Form eines, diese Verbindung in situ abgebenden Mittels, z.B. in Form eines Ammoniumsalzes, wie Ammoniumacetat oder Ammoniumhydrogencarbonat, verwendet werden.

Bei der Ringschlussreaktion a), sowie den Kondensationsreaktionen aa) bis ah) zur Herstellung des üblicherweise in situ gebildeten Ausgangsmaterials für die Ringschlussreaktion, handelt es sich um Varianten der Dihydropyridinsynthese von Hantzsch. Bei der Variante aa) werden insgesamt drei Moleküle Wasser abgespalten; bei weiteren Varianten tritt teilweise an die Stelle der Wasserabspaltung eine Additionsreaktion, d.h. die Wasserabspaltung erfolgt schon bei der Herstellung von einem oder von zwei Ausgangsstoffen. Bei der Umsetzung von Verbindungen der Formel III mit Verbindungen der Formeln XI und X gemäss der Stufe ac), von Verbindungen der Formel X mit Verbindungen der Formel XV gemäss der Stufe ag), oder von Verbindungen der Formel XI mit Verbindungen der Formel XVI gemäss Stufe ah), wird zusätzlich zu bzw. anstelle von Wasser Ammoniak abgespalten. Falls nach der Variante aa) Verbindungen der Formel I hergestellt werden sollen, in denen sich $R_2$ und $R_3$ voneinander unterscheiden, können Nebenprodukte entstehen, die in 2- und

6-Stellung die gleichen Substituenten enthalten. Durch nicht-gleichzeitiges Zusammengeben der Reaktionsteilnehmer kann die Bildung solcher Nebenprodukte indessen herabgesetzt werden, indem man einen bestimmten Reaktionsverlauf fördert, der in situ gemäss einer andern Variante verläuft, da entsprechend der gestaffelten Zugabe der Reaktionskomponenten z.B. zunächst eine Verbindung der allgemeinen Formel X oder der Formel XI entstehen kann.

Die verfahrensgemässen Ringschluss- bzw. Kondensationsreaktionen werden in an sich bekannter Weise durchgeführt, falls notwendig, in Gegenwart eines Kondensationsmittels, insbesondere eines basischen Kondensationsmittels, wie eines Ueberschusses einer basischen Reaktionskomponente oder einer zusätzlichen, z.B. organischen Base, wie Piperidin oder Aethyl-diisopropyl-amin, oder eines Metallalkoholats, wie Alkalimetall-niederalkanolats, und/oder eines geeigneten Dehydratisierungs- oder Wasser-aufnehmenden Mittels, ferner üblicherweise in Gegenwart eines inerten organischen Lösungsmittels und bei Reaktionstemperaturen im Bereich von etwa Raumtemperatur bis etwa 150°C, insbesondere bei Siedetemperatur des Lösungsmittels. Gegebenenfalls erfolgt die Umsetzung in einer Inertgas-, z.B. Stickstoffatmosphäre, und/oder, z.B. bei Verwendung eines niedrigsiedenden Lösungsmittels und/oder von Ammoniak, im geschlossenen Gefäss unter erhöhtem Druck.

Die in den Verfahrensvarianten verwendeten Ausgangsstoffe sind bekannt oder können nach an sich bekannten Verfahren hergestellt werden.

So lassen sich z.B. Ausgangsstoffe der Formel IX durch Umsetzung von Verbindungen der Formel

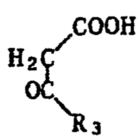

(IXa)

mit Verbindungen der Formel

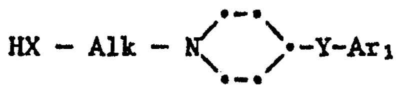

(IXb)

auf übliche Weise herstellen. Anstelle von Carbonsäuren der Formel IXa können auch funktionelle Derivate davon, wie entsprechende Carbonsäureanhydride, insbesondere gemischte Anhydride, wie solche mit Niederalkancarbonsäuren, etwa Ameisensäure, ferner Säurehalogenide, z.B. entsprechende Chloride, Bromide, ferner Säureazide, weiter aktivierte Ester, z.B. Cyanomethylester, verwendet werden. Diese können, gegebenenfalls in Gegenwart von Kondensationsmitteln, durch Umsetzung mit einer Verbindung der Formel IXb, freie Carbonsäuren der Formel IXa auch durch Umsetzen mit Verbindungen der Formel IXb, worin anstelle der Gruppe HX- die Azidogruppe steht, zu Verbindungen der Formel IX umgesetzt werden. Carbonsäuren der Formel IXa können auch als Salze, insbesondere als Alkalimetall- oder Erdalkalimetallsalze mit reaktionsfähigen Estern von Alkoholen der Formel IXb, worin X für Sauerstoff steht, wie entsprechenden Halogeniden, z.B. Chloriden, Bromiden oder Jodiden, oder organischen Sulfonsäureestern, z.B. Niederalkansulfonsäure- oder Arensulfonsäureestern, wie Methansulfonsäure- bzw. p-Toluolsulfonsäureestern, zu entsprechenden Carbonsäureestern umgesetzt werden, oder man hydrolysiert entsprechende hydrolysierbare Iminoester, wie entsprechende Iminoniederalkylester, zu den Estern. Iminoester dieser Art sind beispielsweise aus den Verbindungen der Formel IXa entsprechenden Nitrilen der Formel

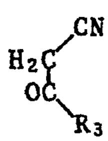

(IXc)

durch Umsetzung mit Verbindungen der Formel IXb, worin X für Sauerstoff steht, in Gegenwart eines sauren Kondensationsmittels, etwa Chlorwasserstoff, in einem geeigneten Lösungsmittel, etwa eines solchen inerten Charakters, wie eines Aromaten, z.B. Benzol, auf übliche Weise zugänglich.

Verbindungen der Formel IXb wiederum sind auf an sich bekannte Weise zugänglich, z.B. durch Umsetzung von Verbindungen der Formel

HX-Alk-A (IXd),

worin A eine geeignete Abgangsgruppe, z.B. eine reaktionsfähige veresterte Hydroxygruppe, wie etwa Halogen, z.B. Chlor, Brom oder Jod oder eine Sulfonyloxygruppe, z.B. eine Arylsulfonyloxy-, wie p-Toluolsulfonyloxygruppe, darstellt, mit Verbindungen der Formel

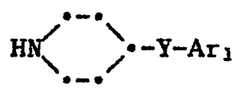
(IXe),

zweckmässigerweise in Gegenwart eines basischen Kondensationsmittels, wie eines Oxids, Hydroxids oder Carbonats eines Alkalimetall- oder Erdalkalimetalls, wie Natriumhydroxid oder Calciumcarbonat, üblicherweise in Gegenwart eines Lösungsmittels, etwa eines Niederalkanols, wie Aethanol, und bei erhöhter oder erniedrigter Temperatur. Verbindungen der Formel IXe können auch in Form ihrer Metallderivate eingesetzt werden, worin das am Stickstoff stehende Wasserstoffatom durch ein geeignetes Metall, etwa Lithium oder Kalium ersetzt ist. In solchen Fällen erfolgt die beschriebene Umsetzung mit Verbindungen der Formel IXd in einem inerten wasserfreien Lösungsmittel, etwa eines solchen ätherartigen Charakters, wie Tetrahydrofuran, oder eines aromatischen Lösungsmittels, etwa Toluol.

Der Formel IXe entsprechende Metallverbindungen sind in üblicher Weise, z.B. durch Umsetzung mit einer geeigneten Alkalimetall-organischen Verbindung, etwa Butyllithium, in einem wasserfreien Lösungsmittel, wie Tetrahydrofuran, zweckmässigerweise unter einem Schutzgas, z.B. Argon, zugänglich, wobei die im Reaktionsgemisch enthaltene Metallverbindung, ohne diese zu isolieren, für die oben beschriebene Umsetzung verwendet werden kann.

Verbindungen der Formel IXe wiederum sind auf an sich bekannte Weise herstellbar, etwa aus solchen Ausgangsstoffen, die anstelle des Piperidinringes eine geeignete, in einen Piperidinring überführbare Gruppe enthält. Solche Gruppen sind etwa solche, die z.B. mittels Reduktion oder mittels ringschliessender Kondensation den Piperidinring bilden, etwa ein entsprechendes 2-Piperidon, welches mittels eines geeigneten Reduktionsmittels, wie Lithiumaluminiumhydrid, in einen Ausgangsstoff der Formel IXe umgewandelt werden kann. Oder man geht z.B. von einem entsprechenden 1,5-Di-halogenpentan, etwa 1,5-Dibrompentan aus, welches mittels Ammoniak, gegebenenfalls im geschlossenen Gefäss und unter Druck, einen Ausgangsstoff der Formel IXe ergibt.

Die Umsetzung von freien Carbonsäuren der Formel IXa mit Verbindungen der Formel IXb erfolgt vorteilhaft in Gegenwart eines sauren, die Wasserabspaltung fördernden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. von Bortrifluorid-Aetherat, in einem Ueberschuss des eingesetzten Alkohols und/oder in einem inerten Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl-, N,N'-Dicyclohexyl-oder N-Aethyl-N'-(3-dimethylaminopropyl)-carbodiimid, in inerten organischen Lösungsmitteln durchführen. Gemischte Anhydride, insbesondere Säurehalogenide, werden beispielsweise in Gegenwart säurebindender Mittel, z.B. organischer, insbesondere tertiärer Stickstoffbasen, wie Triäthylamin, Aethyldiisopropylamin oder Pyridin, oder auch anorganischer Basen, z.B. Alkalimetall- oder Erdalkalimetallhydroxiden oder -carbonaten, wie Natrium-, Kalium-oder Calciumhydroxid bzw. -carbonat, mit Alkoholen oder mit Alkoholaten, z.B. Alkalimetall-niederalkanolaten, umgesetzt.

Die Umsetzungen von reaktionsfähigen Estern, z.B. Cyanmethyl- oder Pentachlorphenylestern, mit Verbindungen der Formel IXb werden beispielsweise in einem gegenüber den Reaktionsteilnehmern inerten Lösungsmittel im Temperaturbereich von etwa 0°C bis etwa 120°C, vorzugsweise bei Raumtemperatur bis etwa 60°C, durchgeführt.

Die Hydrolyse von Imidoesterausgangsstoffen erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Iminoester-salze, z.B. -hydrochloride, nach Zusatz von Wasser direkt zu den entsprechenden Estern hydrolysieren kann. Man kann z.B. auch aus einem Gemisch von Nitril, Alkohol und Schwefelsäure mit geeignetem Wassergehalt ohne Isolierung des in situ entstandenen Imidoesters die gewünschte Esterverbindung der Formel IX erhalten.

Ausgangsstoffe der Formeln XI, XIII, XIV und XV können ausgehend von entsprechenden Ausgangsmaterialien auf analoge und übliche Weise hergestellt werden.

Ausgangsstoffe der Formel IV werden analog wie bei der Herstellung von Ausgangsstoffen der Formel II beschrieben in situ gebildet, und der verfahrensgemässe Ringschluss zu den Endprodukten der Formel I kann unter den für die Herstellung des Ausgangsmaterials gegebenen Bedingungen im gleichen Reaktionsansatz erfolgen. Dementsprechend sind Ausgangsstoffe der Formel IV durch Umsetzung von Verbindungen der Formel IX mit solchen der Formel VIII und Ammoniak, oder von Verbindungen der Formel IX mit solchen der Formel X, oder von Verbindungen der Formel XI mit solchen der Formel VIII oder X zugänglich, wobei solche Umsetzungen üblicherweise in einem geeigneten Lösungsmittel, etwa einem Niederalkanol, wie Aethanol, gegebenenfalls bei erhöhter oder erniedrigter Temperatur, zweckmässigerweise unter einem Schutzgas, wie Stickstoff, durchgeführt werden.

Ausgangsstoffe der Formel V können entsprechend dem bzw. den in ihnen enthaltenen Rest(en) $Ac^o$ und/oder $Ac_1^o$, beispielsweise Carbonsäuren ($Ac^o$ und/oder $Ac_1^o$ ist Carboxyl), Carbonsäureanhydride, insbesondere gemischte Anhydride, wie Säurehalogenide, z.B. -chloride oder -bromide, oder Azide ($Ac^o$und/oder $Ac_1^o$ ist Halogencarbonyl, z.B. Chlor-oder Bromcarbonyl oder Azidocarbonyl), weiter aktivierte Ester, z.B. Cyanmethylester ($Ac^o$ und/oder $Ac_1^o$ ist Cyanmethoxycarbonyl) sein. Diese können, gegebenenfalls in Gegenwart von Kondensationsmitteln, durch Behandeln mit einem entsprechenden Alkohol, in die entsprechenden Ester umgewandelt werden, beispielsweise zur Umwandlung der Gruppe $Ac^o$ in eine der Gruppe $-COOR_1$ entsprechende Carboxylestergruppe durch Umsetzung mit einem der Bedeutung von $R_1$ entsprechenden unsubstituierten oder substituierten Niederalkanol, oder einem reaktionsfähigen Derivat davon, z.B. einem entsprechenden Alkoholat, freie Carbonsäuren auch durch Umsetzen mit geeigneten Diazo-Verbindungen, wie unsubstituierten oder substituierten Diazoniederalkanen, wobei Verbindungen der Formel I entstehen, welche die Gruppe $-COOR_1$ enthalten.

Zur Umwandlung der Gruppe $Ac_1^o$ in die Gruppe der oben definierten Formel Va setzt man einen Ausgangsstoff der Formel V mit einer Verbindung der Formel IXb in üblicher Weise um. Carbonsäureester der angegebenen Art, worin X für Sauerstoff steht, können ebenfalls erhalten werden, wenn als Ausgangsstoffe Salze, insbesondere Alkalimetall- oder Erdalkalimetallsalze, der freien Carbonsäuren verwendet und diese mit reaktionsfähigen Estern von, den Verbindungen der Formel IXb entsprechenden Alkoholen, worin X Sauerstoff ist, wie entsprechenden Halogeniden, z.B. Chloriden, Bromiden oder Iodiden, oder organischen Sulfonsäureestern, z.B. Niederalkansulfonsäure-oder Arensulfonsäureestern, wie Methansulfonsäure- bzw. p-Toluolsulfonsäureestern, behandelt werden, oder wenn man entsprechende hydrolysierbare Iminoester, wie entsprechende Iminoniederalkylester, zu den Estern hydrolysiert.

Iminoester dieser Art sind z.B. aus Ausgangsstoffen der Formel V, worin $Ac^{o}$ und/oder $Ac_{1}^{o}$ die Cyangruppe darstellt, durch Umsetzung mit einem der Bedeutung von $R_1$ entsprechenden Niederalkanol und/oder einem Alkohol der Formel IXb, worin X Sauerstoff ist, in Gegenwart eines sauren Kondensationsmittels, wie Chlorwasserstoff oder konz. Schwefelsäure zugänglich.

Die Umsetzung von freien Carbonsäuren mit Alkoholen, wie unsubstituierten oder substituierten Niederalkanolen oder mit Verbindungen der Formel IXb, erfolgt vorteilhaft in Gegenwart eines sauren, die Wasserabspaltung fördernden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. von Bortrifluorid-Aetherat, in einem Ueberschuss des eingesetzten Alkohols bzw. der Verbindung der Formel IXb und/oder in einem inerten Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl-, N,N'-Dicyclohexyl- oder N-Aethyl-N'-(3-dimethylaminopropyl)-carbodiimid, üblicherweise in inerten organischen Lösungsmitteln durchführen. Gemischte Anhydride, insbesondere Säurehalogenide, werden beispielsweise in Gegenwart säurebindender Mittel, z.B. organischer, insbesondere tertiärer Stickstoffbasen, wie Triäthylamin, Aethyldiisopropylamin oder Pyridin, oder auch anorganischen Basen, z.B. Alkalimetall- oder Erdalkalimetallhydroxiden oder -carbonaten, wie Natrium-, Kalium- oder Calciumhydroxid bzw. -carbonat, mit Alkoholen oder mit Alkoholaten, z.B. Alkalimetall-niederalkanolaten, umgesetzt.

Die Umsetzungen von der Formel V entsprechenden reaktionsfähigen Estern, z.B. Cyanmethyl-, Benztriazol-1-yl oder Pentachlorphenylestern, mit der Bedeutung von $R_1$ entsprechenden Niederalkanolen bzw. mit Verbindungen der Formel IXb werden beispielsweise in einem

gegenüber den Reaktionsteilnehmern inerten Lösungsmittel im Temperaturbereich von etwa 0°C bis etwa 120°C, vorzugsweise bei Raumtemperatur bis etwa 60°C, durchgeführt.

Die Hydrolyse von Imidoesterausgangsstoffen, erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Iminoester-salze, z.B. -hydrochloride, nach Zusatz von Wasser direkt zu den entsprechenden Estern hydrolysieren kann. Man kann Ester z.B. auch aus einem Gemisch von Nitril, Alkohol und Schwefelsäure mit geeignetem Wassergehalt ohne Isolierung des in situ entstandenen Imidoesters die gewünschte Esterverbindung der Formel I erhalten.

Ausgangsstoffe der Formel V mit freier Carboxylgruppe $Ac^o$ und/oder $Ac_1^o$ können z.B. erhalten werden, indem man den entsprechenden 2-Cyanäthylester herstellt, wobei man z.B. in einem der vorstehend beschriebenen Verfahren ab) oder ag) eine Verbindung der Formel X einsetzt, in der anstelle der Gruppe $-COOR_1$ eine 2-Cyanäthoxycarbonylgruppe steht; z.B. kann man einen, die Gruppe $R_2$ enthaltenden 3-Aminocrotonsäure-2-cyanäthylester mit den übrigen Reaktionskomponenten umsetzen, und anschliessend die so erhaltene 2-Cyanäthylester-Verbindung unter milden Bedingungen, z.B. mittels wässrigem oder wässerig-niederalkanolischem 1-n. Natriumhydroxid bei Raumtemperatur, zur freien Carbonsäure spalten. Letztere kann, falls notwendig, in an sich bekannter Weise in die gewünschten reaktionsfähigen funktionellen Derivate übergeführt werden.

Die als Ausgangsstoffe für die Verfahrensvariante c) ebenfalls in Betracht kommenden Nitrilverbindungen der Formel V können z.B. analog zu einer der Verfahrensvarianten aa) bis ah) hergestellt werden, indem man Ausgangsstoffe verwendet, die anstelle des Restes $-COOR_1$ bzw. der Gruppe der Formel IXb eine Cyangruppe enthalten, wie beispielsweise anstelle einer Verbindung der Formel X ein, die Gruppe $R_2$ enthaltendes 3-Aminocrotonitril.

Die für die Verfahrensvariante d) benötigten Ausgangsstoffe der Formel VI können auf an sich bekannte Weise hergestellt werden, z.B. analog den in den Verfahrensstufen aa) bis ah) beschriebenen Umsetzungen, wobei anstelle der Verbindungen IX, XI, XIII, XIV oder XV solche Ausgangsmaterialien eingesetzt werden, die anstelle der Gruppe der Formel

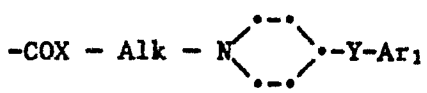

(VIa),

die Gruppe der Formel -COX-Alk-OH (VIb) bzw. einen reaktionsfähigen Ester davon aufweisen. Reaktionsfähige Ester sind z.B. solche mit einer Halogenwasserstoffsäure, z.B. Salzsäure, oder einer organischen Sulfonsäure, wie einer Arylsulfonsäure, z.B. p-Toluolsulfonsäure, gebildete Ester, in denen die Hydroxygruppe z.B. durch Halogen, wie Chlor oder Brom, oder z.B. durch Arylsulfonyloxy, wie p-Toluolsulfonyloxy ersetzt ist. So kann z.B. ein Ausgangsstoff der Formel

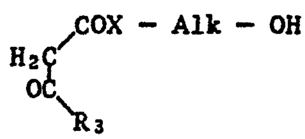

(VIc)

analog den für die Herstellung von Ausgangsstoffen der Formel IX beschriebenen Methoden, z.B. durch Umsetzung von Verbindungen der Formel IXa mit Verbindungen der Formel HX-Alk-OH (VId) oder einem reaktionsfähigen Ester, etwa einem Halogenid, wie Bromid oder Jodid, wie beschrieben, erhalten werden. Oder man setzt ein Nitril der Formel IXc mit einer Verbindung der Formel VId, worin X Sauerstoff ist, in Gegenwart eines sauren Kondensationsmittels, etwa Chlorwasserstoff, zum entsprechenden Iminoester-Salz um, das dann mittels Wasser im sauren Medium, z.B. wie oben beschrieben, zum entsprechenden Carbonsäureester hydrolysiert wird.

Die obigen Reaktionen und ebenso die Verfahrensvariante d) können unter an sich bekannten Reaktionsbedingungen durchgeführt werden, in Ab- oder üblicherweise Anwesenheit von Lösungs- oder Verdünnungsmitteln, je nach Art der Reaktion und/oder Reaktionsteilnehmer bei erniedrigter oder erhöhter Temperatur, z.B. im Temperaturbereich von etwa -10°C bis etwa 150°C, unter atmosphärischem Druck oder in einem geschlossenen Gefäss, gegebenenfalls unter Druck, und/oder in einer inerten Atmosphäre, z.B. unter einer Stickstoffatmosphäre.

Die im Ausgangsmaterial der Formel VII der Verfahrensvariante e) mittels Reduktion in die Gruppe Alk bzw. Y überführbaren Gruppen Z bzw. Y' enthalten Doppel- und/oder Dreifachbindungen und/oder Carbonyl- bzw. Thiocarbonylgruppen im entsprechenden Alkylenrest.

Die Reduktion von ungesättigten Gruppen zur Kohlenstoff-Kohlenstoff-Einfachbindung erfolgt beispielsweise mittels aktiviertem Wasserstoff, wie Wasserstoff in Gegenwart eines Hydrierkatalysators, z.B. eines Nickel-, Platin- oder Palladiumkatalysators, während die Reduktion von Carbonylgruppen zur Methylengruppe mittels Wasserstoff in Gegenwart z.B. eines Kupferchromit-Katalysators oder nach der Methode von Clemmensen, z.B. mittels gegebenenfalls analgamiertem Zink in einer Mineralsäure, etwa Salzsäure, erfolgen kann. Ist die zu reduzierende Carbonylgruppe an ein Stickstoffatom gebunden und liegt somit ein Carboxamid vor, so kann die Reduktion zur Methylengruppe in üblicher Weise, z.B. mit einem Metallhydrid, etwa Lithiumaluminiumhydrid erfolgen. Die Reduktion von Thiocarbonylgruppen kann auf ähnliche Weise erfolgen, z.B. unter Verwendung eines schwefelfesten Katalysators. Die Reduktion der vorstehend genannten Gruppen kann ferner mittels eines Hydridreduktionsmittels, z.B. Natriumborhydrid oder Diboran, erfolgen. Bei diesen Reduktionen muss, sofern erwünscht, Sorge dafür getragen werden, dass andere gegen Reduktion empfindliche Gruppen, z.B. ungesättigte Gruppen oder Nitrogruppen, nicht angegriffen werden, z.B. durch Auswahl des geeigneten Reduktionsmittels, dessen für die durchzuführende Reduktion erforderliche Dosierung und/oder geeigneter Verfahrensbedingungen, z.B. erhöhte oder erniedrigte Temperatur und/oder durch

Anwendung eines geeigneten Lösungsmittels. Diese Umsetzungen werden in an sich bekannter Weise durchgeführt, üblicherweise in Anwesenheit von Lösungs- und Verdünnungsmitteln, je nach Art der Reaktion und/oder Reaktionsteilnehmer bei erniedrigter oder erhöhter Temperatur, z.B. im Temperaturbereich von etwa -15°C bis etwa 120°C unter normalem Druck oder im geschlossenen Gefäss, gegebenenfalls unter Druck und/oder unter einem Schutzgas, etwa Stickstoff.

Ausgangsstoffe der Formel VII können, sofern sie neu sind, nach an sich bekannten Methoden hergestellt werden, z.B. analog den unter a) bis d) beschriebenen Verfahren. So kann man z.B. analog den in den Stufen aa) bis ah) beschriebenen Reaktionsschritten verfahren, wenn man anstelle der Verbindungen IX, XI, XIII, XIV oder XV solche Verbindungen einsetzt, die anstelle der oben definierten Gruppe Va die Gruppe der Formel

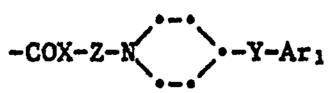

(VIIa)

enthält.

So kann z.B. ein Ausgangsstoff der Formel

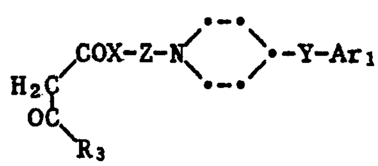

(VIIb),

analog den für die Herstellung von Verbindungen der Formel IX beschriebenen Methoden, etwa durch Umsetzung von Verbindungen der Formel IXa oder reaktionsfähigen Derivaten davon, z.B. Anhydriden, Säurehalogeniden, etwa Chloriden, Aziden, gemischten Estern wie Cyanomethylestern oder Pentachlorphenolestern, verwendet werden. Carbonsäuren der Formel IXa können auch als Salze, insbesondere als Alkalimetall- oder Erdalkalimetallsalze mit reaktionsfähigen Estern der Formel

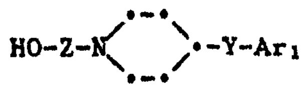 (VIIc),

wie entsprechenden Halogeniden, z.B. Chloriden, Bromiden oder Jodiden, oder organischen Sulfonsäureestern, etwa die mit Niederalkansulfonsäuren, wie Methansulfonsäure, oder Arylsulfonsäuren, wie Benzolsulfonsäure, zu entsprechenden Carbonsäureestern umgesetzt werden. Alkohole der Formel VIIc können andererseits mit Nitrilen der Formel IXc in üblicher Weise z.B. in Gegenwart saurer Kondensationsmittel, wie einer Mineralsäure etwa Chlorwasserstoff- oder Schwefelsäure, zu den Salzen entsprechender Iminoester umgesetzt werden, die dann mittels Wasser, wie beschrieben, zu den Carbonsäureestern hydrolysiert werden. Diese Umsetzungen werden unter üblichen und an sich bekannten Reaktionsbedingungen vorgenommen. Ausgangsstoffe der Formel VIIc wiederum, sind, soweit sie neu sind, auf übliche Weise, z.B. durch Umsetzung von Verbindungen der Formel HO-Z-A (VIId), worin A eine geeignete Abgangsgruppe, z.B. Halogen, wie Chlor, Brom oder Jod, oder eine Sulfonyloxy-, wie p-Toluolsulfonyloxygruppe ist, mit Verbindungen der Formel IXe auf übliche Weise zugänglich.

Verfahrensgemäss erhältliche Verbindungen der Formel I können in an sich bekannter Weise in andere Verbindungen der Formel I umgewandelt werden, etwa durch Umwandlung von in Verbindungen der Formel I enthaltenen Substituenten in andere von der Formel I umfasste Substituenten.

So kann man z.B. eine veresterte Carboxygruppe $-COOR_1$ durch Umesterung in eine andere Estergruppe überführen. Dabei verwendet man als Reagens vorzugsweise Alkoholverbindungen, die einen Siedepunkt aufweisen, der deutlich über demjenigen des Alkohols der veresterten Gruppe in der umzuwandelnden Verbindung der Formel I liegt, und führt die Reaktion z.B. in einem Ueberschuss der Alkoholverbindung und/oder einem inerten organischen, vorzugsweise ebenfalls deutlich über dem Alkohol der veresterten Gruppe siedenden Lösungsmittel, vorzugsweise in Gegenwart eines Katalysators, z.B. eines Alkalimetall-niederalkanolats, wie Natrium- oder Kalium-methanolat oder -äthanolat, in der Wärme und üblicherweise unter Abdestillieren des freigesetzten Alkohols durch.

Ferner kann man Verbindungen der Formel I, in welchen R geeignete azaheterocyclische Gruppen darstellen, deren Ringstickstoffatome N-oxidiert werden können, in entsprechende N-Oxide umwandeln. Die Oxidation kann in an sich bekannter Weise durchgeführt werden, z.B. durch Behandeln mit organischen Persäuren, wie Niederalkanpersäuren oder Arenpersäuren, wie gegebenenfalls geeignet substituierten Perbenzoesäuren, z.B. Peressig- oder 3-Chlorperbenzoesäure, vorzugsweise bei Zimmertemperatur oder etwas darüber liegender Reaktionstemperatur, oder mit wässrigem Wasserstoffperoxid, z.B. bei Temperaturen von bis zu 100°C, in Gegenwart oder Abwesenheit von Niederalkansäuren, z.B. Essigsäure. Dabei muss, insbesondere bei Verwendung von Persäuren, darauf geachtet werden, dass keine Ueberoxidation aufgrund einer zu langen Reaktionsdauer eintreten kann.

Je nach Reaktionsbedingungen können Verbindungen der Formel I freier Form oder in Form von Salzen erhalten werden.

So können erhaltene Säureadditionssalze in an sich bekannter Weise, z.B. durch Behandeln mit einer Base, wie einem Alkalimetallhydroxid, in die freien Verbindungen, oder z.B. durch Behandeln mit geeigneten Säuren oder Derivaten davon in andere Salze umgewandelt werden. Erhaltene freie Verbindungen der Formel I können, z.B. durch Behandeln mit Säuren oder entsprechenden Anionenaustauschern, in ihre Salze umgewandelt werden.

Infolge der engen Beziehung zwischen den Verbindungen der Formel I in freier Form und in Form von Salzen, sind im Vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Verbindungen der Formel I, einschliesslich deren Salze können auch in Form ihrer Hydrate erhalten werden, oder ihre Kristalle können z.B. das zur Kristallisation verwendete Lösungsmittel einschliessen.

Verbindungen der Formel I können, je nach Verfahrensreaktion und/oder Art der Ausgangsstoffe, in Form von Racematgemischen, Racematen oder optischen Antipoden erhalten werden.

Erhaltene Racematgemische können auf Grund der physikalisch-chemischen Unterschiede der Racemate in bekannter Weise in die reinen Racemate bzw. Diastereomeren aufgetrennt werden, beispielsweise durch Chromatographie und/oder fraktionierte Kristallisation.

Racemate lassen sich nach an sich bekannten Methoden in die optischen Antipoden zerlegen, beispielsweise durch Umkristallisation aus einem optisch aktiven Lösungsmittel, mit Hilfe von geeigneten Mikroorganismen oder durch Umsetzen einer Verbindung der Formel I mit salz-bildenden, z.B. basischen, Eigenschaften mit einem optisch aktiven, salzbildenden Mittel, wie einer optisch aktiven Säure, und Trennen der auf diese Weise erhaltenen Gemische von Salzen, z.B. auf Grund ihrer verschiedenen Löslichkeiten, in die diastereomeren Salze, aus denen die Antipoden, z.B. durch Behandeln mit einer Base, freigesetzt werden können.

Optische Antipoden von neutralen Verbindungen der Formel I kann man z.B. auch gemäss Verfahren c) unter Verwendung einer optisch aktiven Säure der Formel V ($Ac^o$ bedeutet Carboxy, $Ac_1^o$ ist verestertes oder amidiertes Carboxy) erhalten, wobei man diese z.B. aus der entsprechenden racemischen Säure in üblicher Weise, z.B. durch Salzbildung mit einer optisch aktiven Base, Trennung der diastereomeren Salze und Freisetzung der optisch aktiven Säure und deren Umwandlung in eine der Formel I entsprechende, die Gruppe $-COOR_1$ enthaltende Verbindung erhält.

Ferner kann man z.B. Verbindungen der Formel I, mit der Gruppe $-COOR_1$ unter Verwendung eines optisch aktiven Alkohols nach dem oben beschriebenen Verfahren umestern und das so erhältliche Diastereomerengemisch, z.B. mittels fraktionierter Kristallisation, in die Antipoden auftrennen.

Vorteilhafterweise isoliert man aus einem Diastereomerengemisch bzw. Racemat das pharmakologisch aktivere Diastereomere bzw. den aktiveren Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivats, z.B. Salzes, und/oder seiner Racemate bzw. Antipoden verwendet oder unter den Reaktionsbedingungen bildet.

Salze von Ausgangsstoffen mit salzbildenden basischen Eigenschaften sind z.B. solche mit Mineralsäuren, wie Salzsäure, Schwefelsäure oder Phosphorsäure, oder mit organischen Säuren, z.B. Essigsäure.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Zwischenprodukte sowie Verfahren zur deren Herstellung bilden ebenfalls einen Gegenstand der vorliegenden Erfindung.

Die Erfindung betrifft ebenfalls die Verwendung der Verbindungen der Formel I oder von pharmazeutisch verwendbaren Salzen von solchen Verbindungen mit salzbildenden Eigenschaften, insbesondere als pharmakologisch, in erster Linie als Coronardilatatoren und Antihypertensiva zur Behandlung von cardiovasculären Krankheitszuständen, wie Angina pectoris und ihre Folgen, Gefässspasmen, hohem Blutdruck und Herzinsuffizienz wirksame Verbindungen. Dabei kann man sie, vorzugsweise in Form von pharmazeutischen Präparaten, in einem Verfahren zur prophylaktischen und/oder therapeutischen Behandlung

des tierischen und menschlichen Körpers, insbesondere zur Behandlung von cardiovasculären Krankheitszuständen, wie Angina pectoris und ihre Folgen, Gefässspasmen, hohem Blutdruck und Herzinsuffizienz verwenden.

Die Dosierung des Wirkstoffs, der allein oder zusammen mit dem üblichen Träger- und Hilfsmaterial verabreicht wird, hängt von der zu behandelnden Spezies, deren Alter und individuellem Zustand, sowie der Verabreichungsweise ab. Die täglichen Dosen liegen z.B. für Mammalien mit einem Körpergewicht von etwa 70 kg, je nach Art der Erkrankung, individuellem Zustand und Alter, vorzugsweise zwischen etwa 10 und 500 mg, insbesondere zwischen etwa 50 mg und etwa 250 mg, und speziell etwa bei 70 mg bis 150 mg bei oraler Verabreichung.

Die Erfindung betrifft im weiteren pharmazeutische Präparate, die Verbindungen der Formel I oder pharmazeutisch verwendbare Salze von solchen Verbindungen mit salzbildenden Eigenschaften als Wirkstoffe enthalten, Verfahren zu ihrer Herstellung, sowie die Verwendung von Verbindungen der Formel I oder deren pharmazeutisch verwendbaren Salze zur Herstellung von Arzneimitteln als Coronardilatoren und Antihypertensiva zur Behandlung von cardiovasculären Krankheitszuständen, wie Angina pectoris und ihre Folgen, Gefässspasmen, zentrale und periphere Durchblutungsstörungen, hohem Blutdruck, Arrythmien und Herzinsuffizienz, ferner zur Anwendung als Hemmer der Plättchenaggregation.

Bei den erfindungsgemässen pharmazeutischen Präparaten handelt es sich um solche zur enteralen, wie peroralen oder rektalen, weiter zur sublingualen, sowie zur parenteralen Verabreichung an Warmblüter. Entsprechende Dosiseinheitsformen, insbesondere zur peroralen und/oder sublingualen Verabreichung, z.B. Dragées, Tabletten oder Kapseln, enthalten vorzugsweise von etwa 10 mg bis etwa 300 mg, insbesondere von etwa 20 mg bis etwa 200 mg einer Verbindung der

Formel I oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinyl-pyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei

ebenfalls Stabilisatoren zugefügt sein können. Bevorzugt sind u.a. Kapseln, die sowohl leicht zerbissen werden können, um durch sublinguale Aufnahme des Wirkstoffes eine möglichst rasche Wirkung zu erzielen, als auch unzerkaut geschluckt werden können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Beispiel 1: Ein Gemisch von 11,85 g 2,6-Dimethyl-4-(3-nitrophenyl)-1,4-dihydropyridin-3,5-dicarbonsäure-methylester-2-chloräthylester, und 10,7 ml Benzylpiperidin wird während 50 Minuten in einem auf 150°C erwärmten Bad unter Argon gerührt. Man löst die Schmelze in einem Gemisch von 100 ml Essigsäureäthylester und 30 ml Wasser, trennt die organische Phase ab, trocknet diese über Natriumsulfat und dampft ein. Der Rückstand wird über Kieselgel mit einem Hexan-Essigsäureäthylester-Methanol-Gemisch (4:2:0,1) chromatographiert und die kristallinen Fraktionen aus 35 ml Acetonitril umkristallisiert, wonach man 2,6-Dimethyl-4-(3-nitrophenyl)-1,4-dihydropyridin-3,5-dicarbonsäure-methylester-2-(4-benzyl-piperidino)-äthylester erhält. Mittels einer ätherischen Lösung von Chlorwasserstoff erhält man daraus das Hydrochlorid, welches nach dem Umkristallisieren aus Dimethoxyäthan bei 185-188° unter Gasentwicklung schmilzt.

Beispiel 2: Ein Gemisch von 2,6-Dimethyl-4-(3-nitrophenyl)-1,4-dihydropyridin-3,5-dicarbonsäure-methylester-2-chloräthylester, 15,8 g 4-(2-Phenäthyl)-piperidin und 6 ml 1,8-Diazabicyclo[5,4,0]undec-7-en in 100 ml Dioxan wird während 21 Stunden unter Rückfluss gekocht. Nach dem Eindampfen am Rotationsverdampfer wird der Rückstand in einem Gemisch von 250 ml Essigsäureäthylester und 100 ml Wasser gelöst, die organische Phase abgetrennt, mit Wasser gewaschen, über Natriumsulfat getrocknet und zur Trockene abgedampft. Der Rückstand wird über Kieselgel (0,040-0,063 mm) mittels Hexan:Essigsäureäthylester-Gemisch (1:1) chromatographiert und der nach dem Aufarbeiten erhaltene 2,6-Dimethyl-4-(3-nitrophenyl)-1,4-dihydropyridin-3,5-dicarbonsäure-methylester-2-[4-(2-phenäthyl)-piperidino]-äthylester mit ätherischer Chlorwasserstofflösung in das Hydrochlorid überführt, welches nach dem Umkristallisieren aus Dimethoxyäthan bei 180-183° unter Gasentwicklung schmilzt.

Beispiel 3: Analog den in der Beschreibung beschriebenen und den in den Beispielen illustrierten Verfahren können, ausgehend von entsprechenden Ausgangsstoffen oder deren Salzen auch folgende Verbindungen der Formel I oder deren Salze, insbesondere deren pharmazeutisch verwendbaren, nicht-toxischen Säureadditionssalze hergestellt werden:

a) 2,6-Dimethyl-4-(3-nitrophenyl)-1,4-dihydropyridin-3,5-dicarbonsäure-methylester-3-(4-benzyl-piperidino)-propylester;

b) 2,6-Dimethyl-4-(3-nitrophenyl)-1,4-dihydropyridin-3,5-dicarbonsäure-methylester-3-[4-(2-phenäthyl)-piperidino]-propylester;

c) 2,6-Dimethyl-4-(3-nitrophenyl)-1,4-dihydropyridin-3,5-dicarbonsäure-methylester-2-[4-(p-fluorbenzyl)-piperidino]-äthylester;

d) 2,6-Dimethyl-4-(3-nitrophenyl)-1,4-dihydropyridin-3,5-dicarbonsäure-methylester-3-[4-(p-fluorbenzyl)-piperidino]-propylester;

e) 2,6-Dimethyl-4-(3-nitrophenyl)-1,4-dihydropyridin-3,5-dicarbonsäure -methylester-2-[4-[2-(p-fluorphenäthyl)]-piperidino]-äthylester;

f) 2,6-Dimethyl-4-(3-nitrophenyl)-1,4-dihydropyridin-3,5-dicarbonsäure-methylester-2-[4-(3-phenylpropyl)-piperidino]-äthylester;

g) 2,6-Dimethyl-4-(3-nitrophenyl)-1,4-dihydropyridin-3,5-dicarbonsäure-methylester-3-[4-(3-phenylpropyl)-piperidino]-propylester;

h) 2,6-Dimethyl-4-(3-nitrophenyl)-1,4-dihydropyridin-3,5-dicarbonsäure-methylester-2-[4-[3-(p-fluorphenyl)-propyl)-piperidino]-äthylester;

i) 2,6-Dimethyl-4-(3-nitrophenyl)-1,4-dihydropyridin-3,5-dicarbonsäure-methylester-2-[4-(p-methoxybenzyl)-piperidino]-äthylester.

Beispiel 4: Tabletten enthaltend 20 mg an aktiver Substanz werden in folgender Zusammensetzung in üblicher Weise hergestellt:

Zusammensetzung:

| | |
|---|---|
| 2,6-Dimethyl-4-(3-nitrophenyl)-1,4-dihydropyridin-3,5-dicarbonsäure-methylester-2-(4-benzylpiperidino)-äthylester-hydrochlorid | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 5: Tabletten enthaltend 1 mg an aktiver Substanz werden in folgender Zusammensetzung in üblicher Weise hergestellt:

Zusammensetzung:

| | |
|---|---|
| 2,6-Dimethyl-4-(3-nitrophenyl)-1,4-dihydropyridin-3,5-dicarbonsäure-methylester-2-(4-benzyl-piperidino)-äthylester-hydrochlorid | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Siueb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 g Gewicht mit Bruchkerbe verpresst.

Beispiel 6: Kapseln enthaltend 10 mg aktiver Substanz werden wie folgt auf übliche Weise hergestellt:

Zusammensetzung:

| | |
|---|---|
| 2,6-Dimethyl-4-(3-nitrophenyl)-1,4-dihyrdo-pyridin-3,5-dicarbonsäure-methylester-2-(4-benzyl-piperidino)-äthylester-hydrochlorid | 2500 mg |
| Talkum | 200 mg |
| Kolloidale Kieselsäure | 50 mg |

Herstellung:
Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt

Beispiel 7:
Anstelle der in den Beispielen 4 bis 6 als aktive Substanz verwendeten Verbindung können auch der 2,6-Dimethyl-4-(3-nitrophenyl)-1,4-dihydropyridin-3,5-dicarbonsäure-methylester-2-[4-(2-phenäthyl)-piperidino]-äthylester sowie die im Beispiel 3 genannten Verbindungen oder deren pharmazeutisch annehmbaren, nichttoxischen Säureadditionssalze als Wirkstoffe in Tabletten, Dragées, Kapseln etc. verwendet werden.

Patentansprüche

1. Verbindungen der Formel

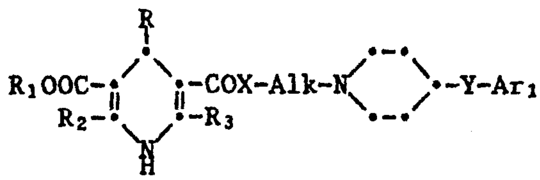

(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyan oder Amino steht, X Sauerstoff oder die Gruppe -NH- darstellt, und Alk Niederalkylen bedeutet, das die Gruppe X vom Ringstickstoffatom durch mindestens zwei Kohlenstoffatome trennt, Y für Alkylen mit 1 bis 3 Kohlenstoffatomen steht und $Ar_1$ einen unsubstituierten ode substituierten Phenylrest darstellt, sowie Salze solcher Verbindungen.

2. Verbindungen der Formel I, in welchen R für einen mono- oder bicyclischen, carbocyclischen Arylrest oder für einen fünf- oder sechsgliedrigen, ein bis und mit vier Ringstickstoffatome, ein Ringsauerstoff- oder Ringschwefelatom, oder ein oder zwei Ringstickstoffatome zusammen mit einem Ringsauerstoff- oder einem Ringschwefelatom als Ringglieder enthaltenden, monocyclischen Heteroarylrest steht, der über ein Ringkohlenstoffatom mit dem Kohlenstoffatom der 4-Stellung des 1,4-Dihydropyridinrings verbunden ist, und der gegebenenfalls einen ankondensierten Benzoring enthält, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzoxadiazolyl, Benzofuranyl, Benzofurazanyl, Benzothienyl, Benzthiazolyl, 2,1,3-Benzthiadiazolyl, Chinolinyl oder Isochinolinyl bedeutet, wobei in diesen Resten Ringkohlenstoffatome gegebenenfalls durch Niederalkyl, Niederalkenyl, Niederalkinyl,

Niederalkylen, Cycloalkyl, Phenyl und/oder Phenylniederalkyl, wobei Niederalkyl, Phenyl oder Phenylniederalkyl gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkylcarbamoyl und/oder Cyan, und cyclische Reste auch Niederalkyl, das seinerseits wie angegeben substituiert sein kann, als Substituenten enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoylxy, Halogen, Nitro, Amino, N-Niederalkyl-amino, N,N-Diniederalkylamino, N-Niederalkyl-N-phenylniederalkyl-amino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenylxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan enthalten kann, oder durch Hydroxy oder Oxido substituiert sein können, $R_1$ Niederalkyl ist, einer der Reste $R_2$ und $R_3$ für Niederalkyl und der andere für Niederalkyl, Cyan oder Amino steht, X Sauerstoff oder die Gruppe -NH- bedeutet, und Alk

Niederalkylen darstellt, das die Gruppe X vom Ringstickstoffatom durch 2 bis 8 Kohlenstoffatome trennt, Y für Alkylen mit 1 bis 3 Kohlenstoffatomen steht, und $Ar_1$ Phenyl bedeutet, welches gegebenenfalls durch Niederalkyl, Niederalkenyl, Niederalkinyl, Niederalkylen, Cycloalkyl, Phenyl und/oder Phenylniederalkyl substituiert ist, wobei Niederalkyl, Phenyl oder Phenylniederalkyl als Substituenten Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, und cyclische Reste auch Niederalkyl, das seinerseits wie angegeben substituiert sein kann, enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N-Diniederalkylamino, N-Niederalkyl-N-phenylniederalkyl-amino, Niederalkylenamino, Oxoniederalkylenmino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkylcarbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan als Substituenten enthalten kann, oder durch Hydroxy oder Oxido substituiert sein können, sowie Salze solcher Verbindungen.

3. Verbindungen der Formel I, worin R und $Ar_1$ jeweils für Phenyl oder Naphthyl stehen und $Ar_1$ für Phenyl steht,, das gegebenenfalls durch Niederalkyl, Phenyl und/oder Phenylniederalkyl, wobei solche Reste ihrerseits Hydroxy, Niederalkoxy, Halogenniederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogenniederalkoxy, Niederalkenyloxy, Halogenniederalkenyloxy, Niederalkylendioxy, Halogen, Nitro, Amino, N-Niederalkylamino, N,N-Diniederalkylamino, Niederalkanoylamino, Carboxy, Niederalkoxycarbonyl, Carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl substituiert ist, R zudem jeweils für über ein Ringkohlenstoffatom gebundenes Pyrryl, Furyl, Thienyl, Pyridyl, 1-Oxido-pyridyl, Imidazolyl, Benzofurazanyl oder Benzoxadiazolyl stehen kann, wobei solche Reste gegebenenfalls, wie für einen Phenyl- oder Naphthylrest R bzw. $Ar_1$ angegeben, substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten können, $R_1$ Niederalkyl ist, $R_2$ und $R_3$ jeweils Niederalkyl bedeuten, oder eine der Gruppen $R_2$ und $R_3$ Niederalkyl ist und die andere Niederalkyl oder Cyan darstellt, X vorzugsweise Sauerstoff oder die Gruppe -NH-bedeutet, und Alk für die Gruppe $-(CH_2)_n-$ steht, worin n Werte von 2 bis 6 darstellt, und Y für Alkylen mit 1 bis 3 Kohlenstoffatomen steht, sowie Salze solcher Verbindungen .

4. Verbindungen der Formel I, worin R und $Ar_1$ jeweils für Phenyl steht, das gegebenenfalls durch Niederalkyl, z.B. Methyl, Hydroxy, Niederalkoxy, z.B. Methoxy oder Aethoxy, Halogenniederalkoxy, z.B. Difluormethoxy oder 2-Chlor-1,1,2-trifluoräthoxy, Halogenniederalkenyloxy, z.B. 1,2-Dichlor-vinyloxy, Niederalkylendioxy, z.B.

Methylendioxy, Halogen, z.B. Fluor, Chlor oder Brom, Trifluormethyl, Nitro, Niederalkanoylamino, z.B. Acetylamino, gegebenenfalls durch gegebenenfalls verestertes oder veräthertes Hydroxy wie Halogen, z.B. Fluor oder Chlor, oder Niederalkoxy, z.B. Methoxy substituiertes Phenyl oder Phenylniederalkyl, und/oder Cyan substituiert ist, wobei ein Phenylrest R bzw. $Ar_1$ einen oder mehrere, gleiche oder verschiedene Substituenten aufweisen kann, R zudem Pyridyl, z.B. 2-, 3- oder 4-Pyridyl, Furyl, z.B. 2-Furyl, 1-Oxido-pyridyl, z.B. 1-Oxido-3-pyridyl, Thienyl, z.B. 2-Thienyl, Benzofurazanyl, z.B. 4-Benzofurazanyl, bedeuten kann, wobei solche Reste R bzw. $Ar_1$ durch Niederalkyl, z.B. Methyl, oder Halogen, z.B. Fluor oder Chlor, substituiert sein können, $R_1$, $R_2$ und $R_3$ jeweils Niederalkyl bedeuten, X insbesondere für Sauerstoff, ferner für die Gruppe -NH- steht, Alk die Gruppe $-(CH_2)_n-$ darstellt, worin n Werte von 2 bis 4 bedeutet, und Y für Alkylen mit 1 bis 3 Kohlenstoffatomen steht, sowie Salze solcher Verbindungen.

5. Verbindungen der Formel I, worin R für unsubstituiertes oder vorzugsweise durch Niederalkyl, z.B. Methyl, Niederalkoxy, z.B. Methoxy, Halogenniederalkoxy oder Halogenniederalkenyloxy, worin Halogen eine Atomnummer bis und mit 35 hat und insbesondere Fluor oder Chlor ist, z.B. Difluormethoxy, Halogen mit einer Atomnummer bis und mit 35, Trifluormethyl, Nitro und/oder Cyan mono-oder di-substituiertes Phenyl steht, wobei Substituenten die 2- und/oder 3-Stellung einnehmen, $R_1$, $R_2$ und $R_3$ jeweils Niederalkyl bedeuten, X insbesondere für Sauerstoff, ferner für die Gruppe -NH- steht, Alk die Gruppe $-(CH_2)_n-$ darstellt, worin n Werte von 2 bis 4 bedeutet, Y für Alkylen mit 1 bis 3 Kohlenstoffatomen, insbesondere 1 bis 2 Kohlenstoffatomen steht, und $Ar_1$ gegebenenfalls durch Halogen, mit einer Atomnummer bis und mit 35 substituiertes Phenyl bedeutet, sowie Salze solcher Verbindungen.

6. Verbindungen der Formel I, worin R für durch Halogen mit einer Atomnummer bis und mit 35, z.B. Fluor, Chlor oder Brom, mono- oder disubstituiertes oder durch Trifluormethyl, Nitro oder Cyan monosubstituiertes Phenyl steht, wobei Substituenten die 2- und/oder

3-Stellung einnehmen, $R_1$ Niederalkyl, insbesondere Methyl oder Aethyl, und $R_2$ und $R_3$ jeweils Niederalkyl, in erster Linie Methyl, bedeuten, X für Sauerstoff steht, Alk die Gruppe $-(CH_2)_n-$ darstellt, worin n Werte von 2 bis 3 bedeutet, Y für 1,3-Propylen, insbesondere Methylen oder 1,2-Aethylen steht, und $Ar_1$ unsubstituiertes oder durch Halogen mit einer Atomnummer bis und mit 35, z.B. Fluor, substituiertes Phenyl ist, sowie Salze solcher Verbindungen.

7. Verbindungen der Formel 1, worin R für durch Halogen mit einer Atomnummer bis und mit 35, z.B. Fluor, Chlor oder Brom, mono- oder disubstituiertes, oder vorzugsweise durch Nitro, ferner durch Cyan monosubstituiertes Phenyl steht, wobei Substituenten die 2- und/oder 3-Stellung einnehmen, $R_1$ Niederalkyl, insbesondere Methyl oder Aethyl, und $R_2$ und $R_3$ jeweils Niederalkyl, in erster Linie Methyl, bedeuten, X für Sauerstoff steht, Alk die Gruppe $-(CH_2)_n-$ darstellt, worin n die Werte von 2 bis 3 bedeutet, Y für Methylen oder 1,2-Aethylen steht, und $Ar_1$ unsubstituiertes oder durch Halogen mit einer Atomnummer bis und mit 35, besonders durch Fluor, in erster Linie in 4-Stellung substituiertes Phenyl ist, sowie Salze solcher Verbindungen.

8. 2,6-Dimethyl-4-(3-nitrophenyl)-1,4-dihydropyridin-3,5-dicarbonsäure-methylester-2-(4-benzyl-piperidino)-äthylester und dessen Salze.

9. 2,6-Dimethyl-4-(3-nitrophenyl)-1,4-dihydropyridin-3,5-dicarbonsäure-methylester-2-[4-(2-phenäthyl)-piperidino]-äthylester und dessen Salze.

10. 2,6-Dimethyl-4-(3-nitrophenyl)-1,4-dihydropyridin-3,5-dicarbonsäure-methylester-3-(4-benzyl-piperidino)-propylester und dessen Salze.

11. 2,6-Dimethyl-4-(3-nitrophenyl)-1,4-dihydropyridin-3,5-dicarbonsäure-methylester-3-[4-(2-phenäthyl)-piperidino]-propylester und dessen Salze.

12. 2,6-Dimethyl-4-(3-nitrophenyl)-1,4-dihydropyridin-3,5-dicarbonsäure-methylester-2-[4-(p-fluorbenzyl)-piperidino]-äthylester und dessen Salze.

13. 2,6-Dimethyl-4-(3-nitrophenyl)-1,4-dihydropyridin3,5-dicarbonsäure-methylester-3-[4-(p-fluorbenzyl)-piperidino]-propylester und dessen Salze.

14. 2,6-Dimethyl-4-(3-nitrophenyl)-1,4-dihydropyridin-3,5-dicarbonsäure-methylester-2-[4-[2-(p-fluorphenäthyl)]-piperidino]-äthylester und dessen Salze.

15. 2,6-Dimethyl-4-(3-nitrophenyl)-1,4-dihydropyridin-3,5-dicarbonsäure-methylester-2-[4-(3-phenylpropyl)-piperidino]-äthylester und dessen Salze.

16. 2,6-Dimethyl-4-(3-nitrophenyl)-1,4-dihydropyridin-3,5-dicarbonsäure-methylester-3-[4-(3-phenylpropyl)-piperidino]-propylester und dessen Salze.

17. 2,6-Dimethyl-4-(3-nitrophenyl)-1,4-dihydropyridin-3,5-dicarbonsäure-methylester-2-[4-[3-(p-fluorphenyl)-propyl)-piperidino]-äthylester und dessen Salze.

18. 2,6-Dimethyl-4-(3-nitrophenyl)-1,4-dihydropyridin-3,5-dicarbonsäure-methylester-2-[4-(p-methoxybenzyl)-piperidino]-äthylester.

19. Pharmazeutisch verwendbare nicht-toxische Säureadditionssalze von Verbindungen der Ansprüche 1-18.

20. Verbindungen gemäss den Ansprüchen 1-19 mit coronardilatatorischen und antihypertensiven Eigenschaften.

21. Verbindungen gemäss Ansprüchen 1-19 zur Anwendung in einem Verfahren zur therapeutischen Behandlung des menschlichen oder tierischen Körpers.

22. Pharmazeutische Präparate enthaltend Verbindungen der Ansprüche 1-19.

23. Verwendung von Verbindungen gemäss den Ansprüchen 1-19 zur Herstellung von pharmazeutischen Präparaten.

24. Verwendung von Verbindungen gemäss den Ansprüchen 1-19 zur Herstellung von Arzneimitteln als Coronardilitatoren und Antihypertensiva zur Behandlung von cardiosvasculären Krankheitszuständen, wie Angina pectoris und ihre Folgezustände, Gefässspasmen, zentrale und periphere Durchblutungsstörungen, hohem Blutdruck, Arrythmien und Herzinsuffizienz, ferner zur Anwendung als Hemmer der Plättchenaggregation oder zur Behandlung der Migräne.

25. Verfahren zur Herstellung von Verbindungen der Formel I gemäss Anspruch 1 und Salzen davon, dadurch gekennzeichnet, das man

a) eine Verbindung der Formel

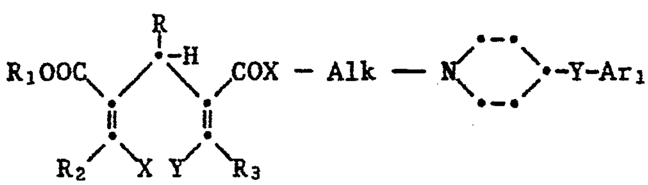

(II),

worin einer der Reste X und Y für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

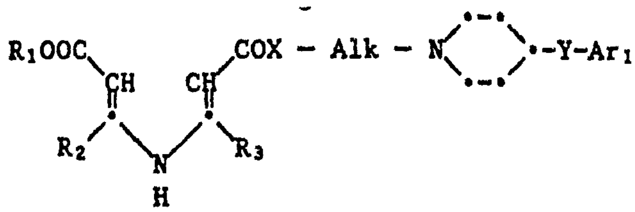 (IV)

oder einem Tautomeren davon oder einem entsprechenden Tautomerengemisch umsetzt, oder

c) in einer Verbindung der Formel

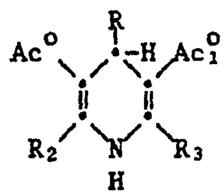 (V),

in welcher einer der Reste $Ac^{o}$ und $Ac_1^{o}$ eine in die Gruppe $-COOR_1$ oder in den Rest der Formel

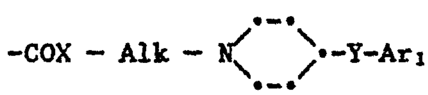 (Va)

überführbare Gruppe bedeutet, und der andere die Gruppe $-COOR_1$ oder die Gruppe der Formel Va bedeutet, den Rest $Ac^{o}$ bzw. $Ac_1^{o}$ in die Gruppe $-COOR_1$ bzw. in einen Rest der Formel Va überführt, oder

d) eine Verbindung der Formel

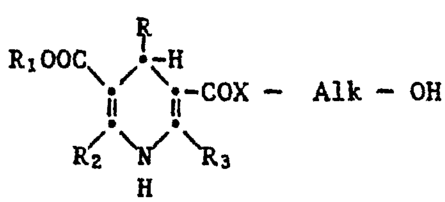 (VI)

oder einen reaktionsfähigen Ester davon, mit einer Verbindung der Formel

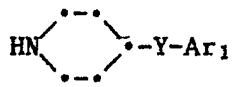

(VIa)

umsetzt, oder

e) eine Verbindung der Formel

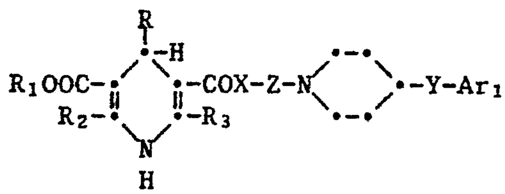

(VII)

worin Z die Bedeutung von Alk hat oder eine mittels Reduktion in die Gruppe Alk überführbare Gruppe darstellt, Y' die Bedeutung von Y hat oder eine mittels Reduktion in die Gruppe Y überführbare Gruppe darstellt, wobei mindestens eine der Gruppen Z und Y' von der Gruppe Alk oder Y verschieden ist, die Gruppe Z und/oder die Gruppe Y' zur Gruppe Alk und/oder zur Gruppe Y reduziert, wobei die Ausgangsstoffe der Formel II bis VII, sofern sie reaktionsfähige Derivate bilden können oder salzbildende Eigenschaften aufweisen, auch in Form von reaktionsfähigen Derivaten oder in Form ihrer Salze verwendet werden können, und die Gruppen R, $R_1$, $R_2$, $R_3$, X, Y, $Ar_1$ und Alk die unter der Formel I angegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder, wenn erwünscht, ein erhaltenes Isomerengemisch in die individuellen Isomeren auftrennt.

26. Die nach dem Verfahren des Anspruches 25 erhältlichen Verbindungen.

FO 7.4 EJA (HM)/hc*eg*cs*bg*